# EUROPEAN PATENT SPECIFICATION

(11) **EP 4 497 141 B1**
(45) Date of publication and mention of the grant of the patent: **25.02.2026**
(21) Application number: 23717324.0
(22) Date of filing: 21.03.2023
(51) Int. Cl.: A61M 5/14, G16H 20/17, G16H 40/63, A61M 5/168, A61M 5/145

(54) **DEVICE, METHOD, AND SYSTEM FOR ACCURATE DELIVERY OF FLUSH INFUSION**
VORRICHTUNG, VERFAHREN UND SYSTEM ZUR GENAUEN ABGABE EINER SPÜLINFUSION
DISPOSITIF, PROCÉDÉ, ET SYSTÈME DE DISTRIBUTION EXACTE DE PERFUSION DE RINÇAGE

(30) Priority: 22.03.2022 US 202263322624 P
(43) Date of publication of application: 29.01.2025
(73) Proprietor: CareFusion 303, Inc., San Diego, CA 92130 (US)
(72) Inventor: DIGGETT, Lisa, San Diego, California 92130 (US); WORKMAN, Michael K., San Diego, California 92130 (US); LANGAN, John, San Diego, California 92130 (US)
(74) Representative: Zenz Patentanwälte Partnerschaft mbB
(86) International application number: PCT/US2023/015830
(87) International publication number: WO 2023/183343

(56) References cited:
- EP-A1- 3 662 945
- EP-A1- 3 965 112

## Description

### TECHNICAL FIELD

The present disclosure is related generally to delivery of a medication and a flush from a syringe pump.

### BACKGROUND

The infusion of medical fluids, such as parenteral fluids, into the human body is accomplished in many cases by means of a syringe pump in which a syringe containing the parenteral fluid is mounted. Syringe pumps typically secure the barrel in a fixed position and utilize a drive head to push or "drive" the plunger into the barrel at a controlled rate to expel the fluid. When a drug is administered to a patient through a syringe, once the pump has pressed the plunger to the bottom of the syringe barrel, no further pressure can be applied. However, there may still be medication in the infusion tubing that needs to be delivered to the patient. Typically, a second infusion will be administered from the same pump using a syringe loaded with a flush fluid such as saline. As the flush fluid is pumped into the tubing, any residual medication from the first syringe will be delivered to the patient.
EP 3 965 112 A1 discloses a medical error reduction system comprising: a drug library editing software for use in creating and revising at least one drug library, the at least one drug library containing a plurality of entries, each of the at least one drug library for use with at least one medical device, the drug library editing software to be executed by a server; at least one drug library database; at least one medical data database; at least one editor computer, each of the at least one editor computer comprising a processor in communication with a user interface, the user interface for use by a user to edit the at least one drug library, the at least one editor computer, at least one drug library database, and at least one medical data database configured to communicate via a network; and wherein while editing the at least one drug library, the user may use the drug library editing software to access medical data.
EP 3 662 945 A1 discloses a peristaltic pump, and a related system method provided. The peristaltic pump includes a cam shaft, first and second pinch-valve cams, first and second pinch-valve cam followers, a plunger cam, a plunger-cam follower, a tube receiver, and a spring-biased plunger. The first and second pinch-valve cams are coupled to the cam shaft. The first and second pinch-valve cam followers each engage the first and second pinch-valve cams, respectively. The plunger cam is coupled to the cam shaft. The plunger-cam follower engages the plunger cam. The tube receiver is configured to receive a tube. The spring-biased plunger is coupled to the plunger-cam follower such that the expansion of the plunger cam along a radial angle intersecting the plunger-cam follower as the cam shaft rotates pushes the plunger cam follower towards the plunger and thereby disengages the spring-biased plunger from the tube. A spring coupled to the spring-biased plunger biases the spring-biased plunger to apply the crushing force to the tube.

### SUMMARY

The invention is defined in the independent claims. Preferable embodiments are further laid out in the dependent claims. The subject technology provides an infusion system comprising: a syringe pump comprising a receptacle for receiving a syringe; one or more sensors configured to generate sensor data pertaining to the syringe within the receptacle of the syringe pump; and one or more processors configured to execute instructions and perform operations, comprising: receiving, based on the sensor data, an indication of a new syringe being loaded into the syringe pump; determining, after receiving the indication based on electronically stored information associated with the syringe pump, that the syringe pump is currently associated with a first fluid delivery order for a predetermined amount of a first fluid, and that at least a first portion of the first fluid was previously delivered by the syringe pump according to the first fluid delivery order before the new syringe was loaded into the syringe pump; determining a second portion of the first fluid remaining to be delivered to complete the predetermined amount of the first fluid for the first fluid delivery order; initiating delivery of a second fluid from the new syringe by the syringe pump according to a second fluid delivery order; when an amount of the second fluid delivered from the new syringe satisfies the second portion of the first fluid remaining to be delivered, automatically, without user intervention: electronically indicating the first fluid delivery order as being complete, de-associating the first fluid delivery order from the syringe pump, and associating the syringe pump with the second fluid delivery order. Other aspects include corresponding methods, apparatus, and computer program products for implementation of the corresponding system and its features.

A machine-implemented method comprises: receiving, from one or more sensors associated with a syringe pump, an indication of a new syringe being loaded into the syringe pump; determining, after receiving the indication based on electronically stored information associated with the syringe pump, that the syringe pump is currently associated with a first fluid delivery order for a predetermined amount of a first fluid, and that at least a first portion of the first fluid was previously delivered by the syringe pump according to the first fluid delivery order before the new syringe was loaded into the syringe pump; determining a second portion of the first fluid remaining to be delivered to complete the predetermined amount of the first fluid for the first fluid delivery order; initiating delivery of a second fluid from the new syringe by the syringe pump according to a second fluid delivery order; when an amount of the second fluid delivered from the new syringe satisfies the second portion of the first fluid remaining to be delivered, automatically, without user intervention: electronically indicating the first fluid delivery order as being complete, de-associating the first fluid delivery order from the syringe pump, and associating the syringe pump with the second fluid delivery order. Other aspects include corresponding systems, apparatus, and computer program products for implementation of the corresponding method and its features.

While the methods and systems disclosed herein are described with regard to syringe pumps, the subject technology is applicable to all infusion pumps. For example, the methods are capable of detecting whether a container volume supplying an infusion fluid (e.g., the medication) is empty. It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description of Implementations below, in conjunction with the following drawings. Like reference numerals refer to corresponding parts throughout the figures and description.
FIG. 1A depicts an example patient care system that includes a syringe pump mounted to a control unit, according to various aspects of the subject technology.
FIG. 1B depicts a first example syringe pump for use in the patient care system of FIG. 1A, according to various aspects of the subject technology.
FIG. 2 depicts a second example syringe pump, according to various aspects of the subject technology.
FIG. 3 depicts an example of an institutional patient care system of a healthcare organization, according to aspects of the subject technology.
FIG. 4 depicts an example system for automatically programming a medical device, according to aspects of the subject technology.
FIG. 5 depicts an example process for accurate delivery of a flush infusion, according to aspects of the subject technology.
FIG. 6 is a conceptual diagram illustrating an example electronic system for accurate delivery of a flush infusion, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

In today's syringe-based infusion systems, two syringes are treated as two separate orders to the infusion pump with no relationship to each other. This can cause issues with documenting in the patient record when the full drug dose was delivered and completed as well as when the flush (and how much flush) was administered. This is due in part to a lack of documented linkage between the medication order and the flush order as well as a delay between the clinical administration steps.

When a syringe is used to flush an infusion line, a user manually programs the syringe pump to deliver the flush. The system may be completely unaware that the clinician is flushing a medication line. Furthermore, existing systems may only recognize the flush fluid and not appreciate the connection with the flush fluid to the primary medication. For some medications, safe administration of the flush fluid can vary based on the medication being flushed.

The subject technology addresses how to accurately and safely program, administer, and electronically document a flush for a syringe type infusion pump. The subject technology provides syringe flush programming features that include a) electronically accounting volume from primary and flush syringes, b) message timing for acknowledging a flush APR order without necessarily needing to start; and providing linkage between the medication order and the flush order.

The subject technology offers more accurate reporting of volume infused, time of infusion, and infusion status for both medication and flush orders to an electronic medical records (EMR) system, avoiding the appearance of under- or overdosing in a patient's chart. The described features also allow association of the flush order with the medication order. When a new syringe is loaded in a syringe pump, the pump determines at least a first portion of a predetermined amount of a medication was previously delivered by the pump according to a medication delivery order before the syringe was loaded, and determines a second portion of the medication remaining to be delivered to complete the medication delivery order. A delivery of a second fluid (e.g., a flush) is initiated by the syringe pump and, when an amount of the second fluid delivered from the new syringe satisfies the second portion of the medication remaining to be delivered, the pump automatically, without user intervention, indicates the medication delivery order as being complete, de-associates the medication delivery order from the syringe pump, and associates the syringe pump with the second fluid delivery order.

The subject technology further improves medication administration safety for flush orders by generating a programming confirmation with the transmission of any flush auto programming request. Unlike manually programmed infusions, an auto programming request infusion may be evaluated by clinical safety parameters to ensure the right drug, is provided in the right amount, to the right patient, at the right time through the right route.

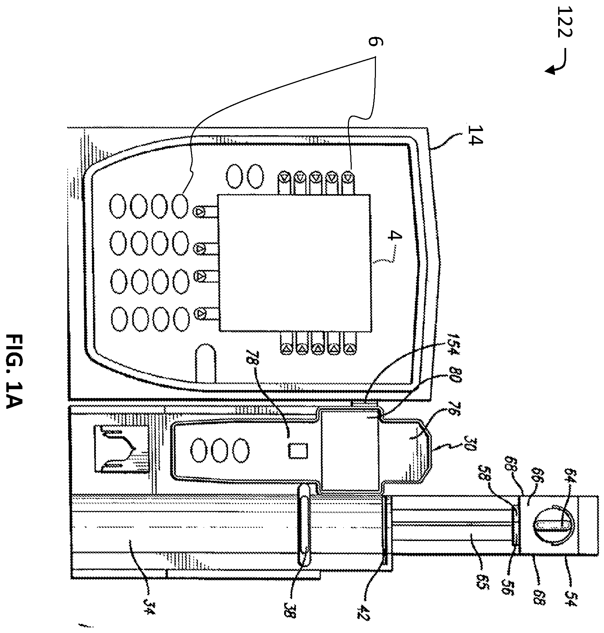

FIG. 1A depicts an example patient care system 122 that includes a syringe pump 30 mounted to a control unit 14, according to various aspects of the subject technology. According to various implementations, the control unit 14 may provide control and monitoring functionality for the syringe pump 30. In this regard, the control unit may include a display 4 for visually communicating various information, such as the operating parameters of a connected pump and alert indications and alert messages, and control keys 6 for selecting and/or setting control parameters and/or options for controlling connected modules such as syringe pump 30. The control unit 30 may also include a speaker to provide audible alerts. In some implementations, the display 4 may be implemented as a touchscreen display. In such implementations, the control keys 6 may be omitted or reduced in number by providing corresponding interactive elements via a graphical user interface presented via the display 4. In some implementations, each control key 6 may select a corresponding option displayed in display 4.

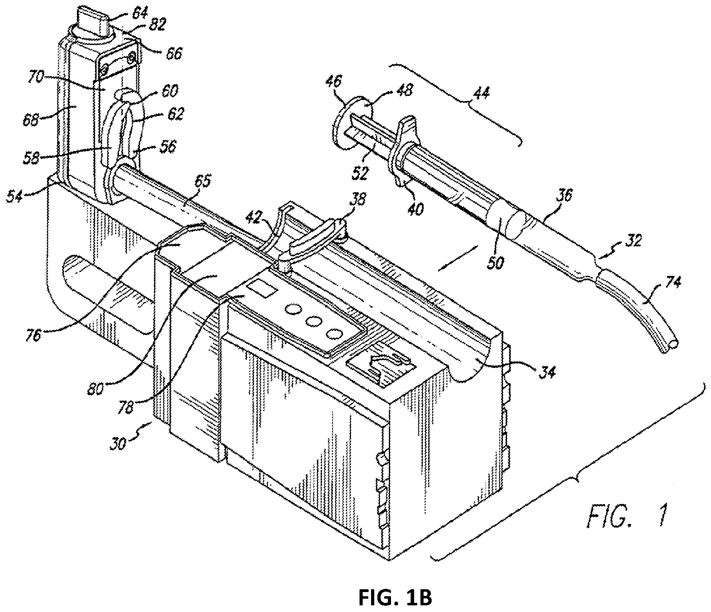

The syringe pump 30 can include a control panel 76 providing multiple buttons 78 for control of the pump 26 as well as a display 80 used to present pump-specific information to the operator (see also FIG. 1B). The buttons 78 can allow the operator to program the pump 26 for the flow rate, the volume to be infused, and other pump parameters. The display 80 can present the programmed flow rate, the amount of fluid remaining to be infused, as well as alarms and other information.

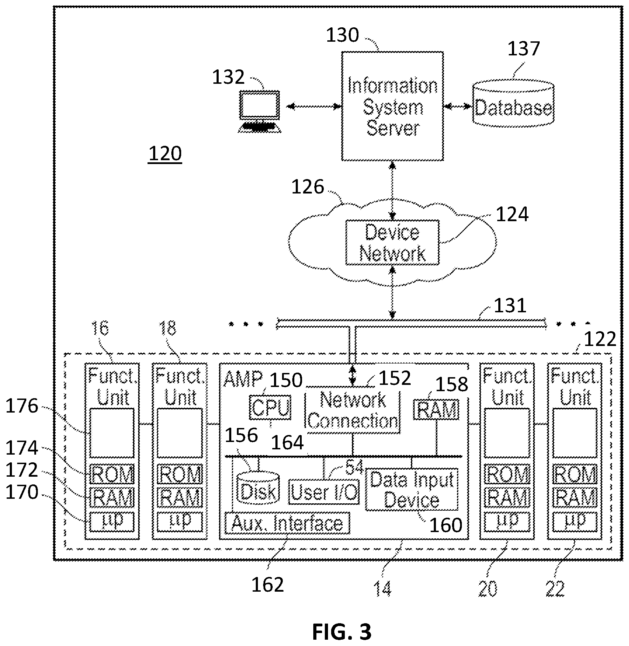

The control unit 14 may include a communications system (not shown) with which the control unit 14 may communicate with external equipment such as a medical facility server or other computer and with a portable processor, such as a handheld communication device or a laptop-type of computer, or other information device that a clinician may have to transfer information as well as to download drug libraries to a control unit (such as pump 30 or, e.g., modules of FIG. 3). The communication module may be used to transfer access and interaction information for clinicians encountering the control unit or device coupled therewith (e.g., pump 30 or bar code scanner). The communications system may include one or more of a radio frequency (RF) system, an optical system such as infrared, a BLUETOOTH^{™} system, or other wired or wireless system. The bar code scanner and communications system may alternatively be included integrally with the infusion pump 30, such as in cases where a control unit is not used, or in addition to one with the control unit 14. Further, information input devices need not be hard-wired to medical instruments, information may be transferred through a wireless connection as well. Additionally, other types of modules may be connected to the pump modules or to the control unit such as a syringe pump module, patient controlled analgesic module, End Tidal CO₂ monitoring module, oximeter monitoring module, or the like.

In some embodiments, the pressure measurements from the upstream and/or downstream pressure sensors are transmitted to a server or other coordination device, and the methods disclosed herein are implemented on the server or other coordination device. For example, a pressure sensor may be used to determine a pressure or force within the infusion line downstream of the pump (e.g., between the patient and the pump). More sophisticated and computationally intensive approaches like machine-learning can be implemented on a server (or on a control unit with a larger memory and/or CPU resources). In some embodiments, machine learning is used to identify empty conditions based on pressure signals received from the pump.

FIG. 1B depicts an example syringe pump infusion system including a first example syringe pump 30, according to various aspects of the subject technology. Syringe pumps can be used for the infusion of medical fluids, such as parenteral fluids, into the human body. In many cases, smooth delivery of medical fluids is desired. For example, where the medical fluid includes a medication, delivery at a consistent or predicable flow rate allows a medical professional to properly plan and execute a treatment. Mechanical interactions between parts of an infusion system can produce resistance to smooth delivery. Aspects of the embodiments discloses herein address these concerns to provide more consistent and predictable flow during driver operation.

According to various implementations, the depicted infusion syringe pump 30 includes a drivetrain subsystem. A syringe 32 is shown next to the pump rather than mounted in the pump, for clarity of illustration. The syringe pump 30 includes a cradle/receptacle 34 in which a barrel 236 can rest when mounted in the syringe pump 30. The cradle 34 can include a clamp 38 to securely hold the barrel 36 in a fixed position in the cradle 34 so that axial and lateral movement is resisted. The clamp 38 can be pivoted so that it may be moved into an open position to permit loading or removal of the syringe 32 and a closed position in which it extends over the cradle 34 to hold a mounted barrel 36. A barrel flange 40 of the syringe 32 can be located in a barrel flange groove 42 in the syringe pump 30 to immobilize the barrel 36 from axial movement during movement of the plunger 44 within the barrel 36.

The syringe 32 can include the barrel 36 and the plunger 44. The plunger 44 can include a push-button 46 having an inner side 48 and being interconnected with a stopper 62 of the plunger 44 by a piston 50. The plunger 44 can include the stopper 62 to sealingly engage an inner wall of the barrel 36 to prevent fluid from leaking past the stopper 62. When mounted in the syringe pump 30, the push-button 46 can be held by a drive head 54 with a plunger retainer comprising a pair of pivotally mounted claws, first retainer claw 56 and second retainer claw 58, shown in the closed position in FIG. 1B. The retainer claws 56 and 58 can curve inwardly toward each other to grasp the push-button 46 when mounted in the syringe pump 30. A rotation knob 64 can be used to control the positions of the first and second retainer claws 56 and 58 to allow removal and insertion of the push-button 46 and to release the split-nut from the driveshaft to permit axial positioning of the drive head 54. Syringes can be provided for use with a syringe pump with different quantities of fluid, and the plunger can be located at different positions in relation to the barrel.

The drive head 54 may allow manual adjustment to accommodate syringes with different beginning plunger positions. A syringe inserted in the cradle 34 can align with the drive head 54 within a particular axial range. The points where the axial center lines of the syringes intersect the driver can change according to the size of the syringe but only in one direction along the drive head 54. A guide device 65 can extend from the drive head 54 to a point within a body of the syringe pump 30.

According to some implementations, the system can include components for controlling advancement of the plunger 44 within the barrel 36 of the syringe 32. The drive head 54, which engages the push-button 46 of the syringe 32, can be moved by a drivetrain (not shown). For example, the pump 30 can include a motor that operates to rotate a lead screw engaged by a screw drive mechanism, such as a split nut, that translates the rotational motion of the lead screw into linear motion. The drive head 54 is connected to the screw drive mechanism and drives plunger 44 into the barrel 36 in accordance with the movement of the lead screw to expel fluid from the barrel 36.

According to some embodiments, the motor may include a stepper motor, a brushed DC electric motor, a brushless DC electric motor, a servo motor, an AC motor, or another type of motor. The drivetrain may include appropriate gears, axles, shafts, chains, hydraulics, and/or any other components for translating rotational motion of the motor into linear motion of the drive head 54. According to some embodiments, the drivetrain, including the motor, can include linear actuating components, such as a linear stepper motor. For example, a linear motor can act directly on the drive head, or a component attached thereto, to control linear motion thereof.

The drive head 54 can interact with the syringe 32 in a manner that facilitates prompt, consistent, and predicable infusion of a fluid from the syringe 32 to the patient. The features described herein can be used individually or in combination to improve the ability of the system to deliver medication promptly and smoothly at low flow rates.

In some implementations, the syringe pump 30 includes a cradle sensor positioned in or near the cradle 34. For example, the cradle sensor may be configured to detect whether a syringe is loaded in the cradle. In some implementations, the syringe pump 30 includes a barrel clamp sensor positioned on or near the clamp 38. For example, the barrel clamp sensor 348 may be configured to detect movement of the clamp 310. As another example, the barrel clamp sensor may be configured to detect whether or how tightly the clamp 38 grips the barrel of the syringe 32 (e.g., corresponding to a size of the syringe). As yet another example, the barrel clamp sensor may be configured to detect a degree of displacement of the clamp. A completely displaced clamp may indicate that the clamp is open (e.g., for loading or unloading of a syringe). By contrast, a clamp displaced only slightly from a completely closed position may indicate that the clamp is gripping a syringe (e.g., with the degree of displacement corresponding to a diameter of the syringe).

In some implementations, the syringe pump 30 includes a drive head sensor positioned on or near the drive head 54. For example, the drive head sensor may be configured to detect movement of the drive head. As another example, the drive head sensor 350 be configured to detect a position of the drive head. The position may be used to detect when the syringe is empty (e.g., the plunger 44 fully inserted). In some implementations, the syringe pump module includes a plunger retainer sensor positioned on or near the retainer claw. For example, the retainer sensor may be configured to detect opening and/or closing of the retainer claw . As another example, the retainer sensor may be configured to detect whether or how tightly the retainer claw grips the push-button of the plunger 44. One or more of the foregoing sensors may be used to detect when a new syringe is being loaded into the syringe pump 30, or when a syringe is being unloaded from the pump. In some implementations, a sensor may be used to optically detect when a syringe is emptied (e.g., by detecting the piston 50 at the end of the barrel 36).

When loading a syringe 32, a clinician releases and raises up the drive head. Such physical interaction may be detected by the drive head sensor . The clinician may then pull out and/or twist the barrel clamp out of the way, activating the barrel clamp sensor. The clinician loads the syringe and the cradle sensor detects the barrel of syringe when its loaded into the cradle/receptacle. A barrel flange sensor detects the flange when the flange is secured by the barrel flange. The clinician twists the barrel clamp back in place to secure the syringe within the cradle/receptacle 34 and barrel clamp sensor may detect the clamp has secured the syringe 32. The clinician opens the retainer claws and lowers the drive head onto the push-button of the plunger, releasing the claws to secure the plunger as the claws close around the push-button. Such physical interaction may be detected by the drive head sensor and retainer sensor, respectively.

As depicted in FIGS. 1A, the syringe pump 30 can be mounted to a control unit 14, together forming a modular patient care system. The control unit may perform various functions for the pump such as programming and communications. Control elements and functions can be included with and performed, at least in part, by the control unit 14. In addition to the syringe pump 30 mounted to the control unit 14, other modules, such as those providing patient monitoring or therapies, can also form part of the patient care system. The control unit 14 can provide a centralized interface for the various attached modules. One or more syringe pumps can be mounted to the control unit 14 and/or each other.

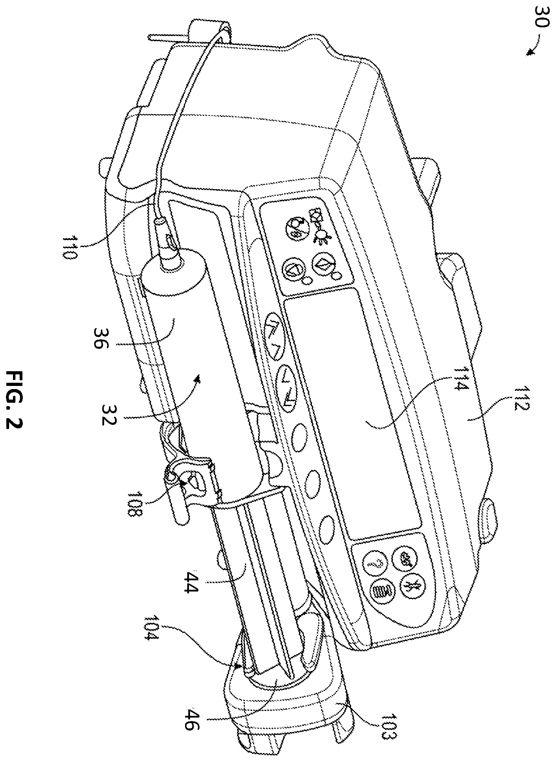

FIG. 2 depicts a second example syringe pump 30 infusion device, according to various aspects of the subject technology. While the example syringe pump 30 is shown as a stand alone device, the syringe pump may be configured as a functional module of a modular infusion system such as a syringe module for the BD Alaris^{™} system.

When a syringe 32 is loaded in the syringe pump 30, a plunger flange (or push button) 46 at the end of a syringe plunger piston 44 is held in or against a plunger drive head 103 by a flange clamp 104. The syringe barrel 36 is secured by a syringe clamp 108. The drive head 103 includes a pushing surface on which the plunger flange 46 will rest as the drive head 103 moves forward toward the syringe barrel 36 pushing the plunger piston 44 into the barrel 36 of the syringe to expel the syringe contents through an administration tubing 110 to the patient. As will be described further, the drive head 103 may be connected to a screw drive mechanism, including a motor, for connecting the linear motion of the screw drive mechanism to the syringe plunger in order to empty the syringe. The rate is controlled by the syringe pump 100 based on programmed parameters (e.g., desired rate, type of syringe).

Syringe pumps do not typically experience any upstream pressure conditions because the fluid to be infused is housed in the syringe barrel 106 and is pushed into an administration set 110 by way of the plunger piston 44. Downstream pressure conditions can be detected by a force sensor housed in or upon a pump system 112. In some implementations, a force sensor measures the force exerted by the drive head 103 of the syringe pump on the syringe plunger piston 44.

In some implementations, the syringe pump 100 may include a high-resolution pressure/force sensor that interfaces with a pressure disc (not shown) on the syringe administration set. The pressure disc provides a relatively large area in contact with the pressure sensor. This allows the pressure sensor to measure the pressure inside the administration set more directly (not through the syringe plunger head) and with higher resolution and higher accuracy compared with the drive head force sensor. The measurements from this pressure sensor and the drive head force sensor can be used independently or in conjunction with each other to detect an empty condition in a syringe pump.

As well as operating buttons or switches, which the operator may use to activate and program the syringe pump 100, there is a display screen 114. The display screen 114 may be an LCD (liquid crystal display) having a small number of segments, for example seven segments in a figure-of-eight configuration per character, adapted to display a small number of alphanumeric characters. The display may be monochromatic, for example, it might only display red, green, or grey/black characters. Alternatively, the display 114 can be a more complicated liquid crystal display capable of displaying more characters or more complicated characters. The LCD may be backlit, for example, using light emitting diodes (LEDs). In some implementations, the infusion pump may include a TFT LCD. A TFT is a thin-film transistor-based LCD technology. In some implementations, the display screen 114 is also a touchscreen such as a capacitive touchscreen.

When programming the syringe pump 30, the user may input the type of syringe being used. The syringe pump 100 may store in an internal memory a database of known syringe types containing information such as syringe diameter and stroke. The infusion pump firmware calculates the position of the syringe plunger and syringe piston based on movement of the syringe drive head and the type and size of the syringe. This allows the machine to display the calculation of volume infused, time elapsed, volume remaining and time remaining. As infusion continues and the drive head moves, these calculations can be updated, and the displayed information changed.

The syringe pump 30 may be provided with an input interface with controls operable to enter, increase or decrease pumping parameters, such as the mass flow rate setting shown on a display, or the VTBI (volume to be infused) setting shown on the display. In some cases, an input key may be physically present on the device (as depicted) or may be graphically displayed in a touchscreen display 114.

In some implementations, the syringe pump 30 may be configured to identify (e.g., using a sensor) a disposable container loaded by the device. The syringe pump 30 of FIG. 2 may include one or more (or all) of the same or analogous sensors as discussed above with regard to FIG. 1B. In this regard, a processor of the syringe pump (e.g., internally or in the control unit 14) may detect when the syringe becomes empty (e.g., by using a drive head sensor, piston sensor, or pressure sensor), and when the user is loading or unloading the syringe from the syringe pump.

The syringe pump 30 may perform electro-mechanical measurements on the loaded syringe to identify certain characteristics about the loaded container. For example, a syringe pump may include a sensor that measures the size of the syringe inserted into the pump, for example, based on how tightly the syringe is being hugged. The clock position may determine the size of the barrel (e.g., whether it is a 6, 10, 50 ml syringe, etc.). Based on the physical measurements made by the pump, the syringe pump may determine a list of possible candidate syringes. The device may then request confirmation via the display whether the container is within that list. During the infusion, volume infused and/or flow rate may be calculated based on the syringe type (e.g., based on the size of the syringe barrel).

FIG. 3 depicts an example of an institutional patient care system 120 of a healthcare organization, according to aspects of the subject technology. A patient care device (or "medical device" generally) 122 is connected to a hospital network 124 via a LAN/WAN 126. The term patient care device or patient care unit may be used interchangeably with a control unit attached to a pump, either which may include various ancillary medical devices such as an infusion pump, a vital signs monitor, a medication dispensing device (e.g., cabinet, tote), a medication preparation device, an automated dispensing device, a module coupled with one of the aforementioned (e.g., a syringe pump module configured to attach to an infusion pump), or other similar devices. Each element 122 is connected to the internal healthcare network 124 by a transmission channel 131. Transmission channel 131 is any wired or wireless transmission channel, for example an 802.11 wireless local area network (LAN). In some implementations, network 124 also includes computer systems located in various departments throughout a hospital. For example, network 124 optionally includes computer systems associated with an admissions department, a billing department, a biomedical engineering department, a clinical laboratory, a central supply department, one or more unit station computers and/or a medical decision support system. As described further below, network 124 may include discrete subnetworks. In the depicted example, network 124 includes a device network by which patient care devices 122 (and other devices) communicate in accordance with normal operations.

Additionally, institutional patient care system 120 may incorporate a separate information system server 130. Moreover, although the information system server 130 is shown as a separate server, the functions and programming of the information system server 130 may be incorporated into another computer, if such is desired by engineers designing the institution's information system. Institutional patient care system 120 may further include one or multiple device terminals 132 for connecting and communicating with information system server 130. Device terminals 132 may include personal computers, personal data assistances, and mobile devices such as laptops, tablet computers, augmented reality devices, or smartphones, configured with software for communications with information system server 130 via network 124.

Patient care device 122 comprises a system for providing patient care, such as the BD Alaris^{™} system. Patient care device 122 may include or incorporate pumps, physiological monitors (e.g., heart rate, blood pressure, ECG, EEG, pulse oximeter, and other patient monitors), therapy devices, and other drug delivery devices may be utilized according to the teachings set forth herein. In the depicted example, patient care device 122 comprises a control unit 14, also referred to as interface unit or programming module, connected to one or more functional modules 16, 18, 20, 22. Control unit 14 includes a central processing unit (CPU) 150 connected to a memory, for example, random access memory (RAM) 158, and one or more interface devices such as user interface device 154, a coded data input device 60, a network connection 152, and an auxiliary interface 162 for communicating with additional modules or devices. Control unit 14 also, although not necessarily, includes a main non-volatile storage unit 156, such as a hard disk drive or non-volatile flash memory, for storing software and data and one or more internal buses 64 for interconnecting the aforementioned elements.

In various implementations, user interface device 154 is a touch screen for displaying information to a user and allowing a user to input information by touching defined areas of the screen. Additionally, or in the alternative, user interface device 154 could include any means for displaying and inputting information, such as a monitor, a printer, a keyboard, softkeys, a mouse, a track ball and/or a light pen (e.g., display 4 of FIG. 1A). Data input device 160 may be a bar code reader capable of scanning and interpreting data printed in bar coded format. Additionally or in the alternative, data input device 160 can be any device for entering coded data into a computer, such as a device(s) for reading a magnetic strips, radio-frequency identification (RFID) devices whereby digital data encoded in RFID tags or smart labels (defined below) are captured by the reader 160 via radio waves, PCMCIA smart cards, radio frequency cards, memory sticks, CDs, DVDs, or any other analog or digital storage media. Other examples of data input device 160 include a voice activation or recognition device or a portable personal data assistant (PDA). Depending upon the types of interface devices used, user interface device 154 and data input device 160 may be the same device. Although data input device 160 is shown to be disposed within control unit 14, it is recognized that data input device 160 may be integral within pharmacy system 34 or located externally and communicating with pharmacy system 34 through an RS-232 serial interface or any other appropriate communication means. Auxiliary interface 162 may be an RS-232 communications interface, however any other means for communicating with a peripheral device such as a printer, patient monitor, infusion pump or other medical device may be used without departing from the subject technology. Additionally, data input device 160 may be a separate functional module, such as modules 16, 18, 20 and 22, and configured to communicate with controller 14, or any other system on the network, using suitable programming and communication protocols.

Network connection 152 may be a wired or wireless connection, such as by Ethernet, WiFi, BLUETOOTH, an integrated services digital network (ISDN) connection, a digital subscriber line (DSL) modem or a cable modem. Any direct or indirect network connection may be used, including, but not limited to a telephone modem, an MIB system, an RS232 interface, an auxiliary interface, an optical link, an infrared link, a radio frequency link, a microwave link or a WLANS connection or other wireless connection.

Functional modules 16, 18, 20, 22 are any devices for providing care to a patient or for monitoring patient condition. At least one of functional modules 16, 18, 20, 22 may be an infusion pump module such as syringe pump 30 (FIG. 1B or 2) for delivering medication or other fluid to a patient. For the purposes of this discussion, functional module 116 is an infusion pump module. Each of functional modules 16, 18, 20, 22 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart rate monitor, an intracranial pressure monitor, or the like. Functional module 16, 18, 20 and/or 22 may be a printer, scanner, bar code reader, near-field communication reader, RFID reader, or any other peripheral input, output or input/output device.

Each functional module 16, 18, 20 and/or 22 communicates directly or indirectly with control unit 14, with control unit 14 providing overall monitoring and control of device 122. Functional modules 16, 18, 20 and/or 22 may be connected physically and electronically in serial fashion to one or both ends of control unit 14. However, it is recognized that there are other means for connecting functional modules with the interface unit that may be utilized without departing from the subject technology. It will also be appreciated that devices such as pumps or patient monitoring devices that provide sufficient programmability and connectivity may be capable of operating as stand-alone devices and may communicate directly with the network without connected through a separate interface unit or control unit 14. As described above, additional medical devices or peripheral devices may be connected to patient care device 122 through one or more auxiliary interfaces 162.

Each functional module 16, 18, 20, 22 may include module-specific components 176, a microprocessor 170, a volatile memory 172 and a nonvolatile memory 174 for storing information. It should be noted that while four functional modules are shown, any number of devices may be connected directly or indirectly to control unit 14. The number and type of functional modules described herein are intended to be illustrative, and in no way limit the scope of the subject technology. Module-specific components 176 include any components necessary for operation of a particular module, such as a pumping mechanism for infusion pump module 116.

While each functional module may be capable of a least some level of independent operation, control unit 14 monitors and controls overall operation of device 122. For example, as will be described in more detail below, control unit 14 provides programming instructions to the functional modules 16, 18, 20, 22 and monitors the status of each module.

Medical devices incorporating aspects of the subject technology may be equipped with a network interface module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, patient care device 122 and network 124 may communicate via automated interaction, manual interaction or a combination of both automated and manual interaction. Automated interaction may be continuous or intermittent and may occur through direct network connection 154, or through RS232 links, MIB systems, RF links such as BLUETOOTH, IR links, WLANS, digital cable systems, telephone modems or other wired or wireless communication means. Manual interaction between patient care device 122 and network 124 involves physically transferring, intermittently or periodically, data between systems using, for example, user interface device 154, coded data input device 160, bar codes, computer disks, portable data assistants, memory cards, or any other media for storing data. The communication means in various aspects is bidirectional with access to data from as many points of the distributed data sources as possible. Decision-making can occur at a variety of places within network 124. For example, and not by way of limitation, decisions can be made in health information system (HIS) server 130, decision support, remote data server , hospital department or unit stations, or within patient care device 122 itself.

All direct communications with medical devices operating on the network 124 in accordance with the subject technology may be performed through information system server 130, known as the remote data server (RDS). In accordance with aspects of the subject technology, network interface modules incorporated into medical devices such as, for example, infusion pumps or vital signs measurement devices, ignore all network traffic that does not originate from an authenticated RDS. The primary responsibilities of the RDS of the subject technology are to track the location and status of all networked medical devices that have NIMs, and maintain open communication.

In some implementations, server 30 may be representative of or include multiple servers. In some implementations, server 30 may include an EMR system (e.g., EMR server 202). According to some implementations, server 30 includes a formulary and/or pharmacy information system. Pharmacy information systems may enable a safer physician medication order process. A pharmacy website (e.g., provided by the server) may provide the physician with a list of available drugs from which the physician may select. The pharmacy website may contain a drug library having the list of available drugs but may also contain and present to the physician the drug names associated with recommended dosages and dose limits that have been established or adopted by the healthcare facility. In such a case where the physician need only select items from the computer screen rather than having to manually type in drug names and drug administration numbers (such as infusion rates, times, etc.) associated with administration of the medication, a more accurate medication process should result.

If a clinical order is for administration of a particular medication regimen, the order will be transmitted to the facility's pharmacy information system 130. The pharmacy reviews the order, and once the order has been prepared, the order may be transmitted to the nurse station for matching with the appropriate patient. Formulary is an approved list of drugs for use (e.g., available to order for a patient) within a medical facility. Within a formulary, there may be indication for use information and/or concentrations and drug ranges approved for the facility. As will be described further, a formulary may be used to define one or more medical device drug libraries, which may then be provided to infusion pumps within a hospital network. Inside the library, there is medication information such as drug names, concentration, diluent volume, strength, minimum or maximum infusion parameters for a drug, and other parameters. The establishment of these parameters, along with parameters for off-formulary orders, via the system 130 is useful for maintaining consistency across the healthcare environment and ensuring an order is intelligible and executed according to expectations by other devices within the system 130 (e.g., an infusion pump).

With further reference to FIG. 3, patient care device 122 is capable of operating in several different modes, or personalities, with each personality defined by a configuration database. The configuration database may be a database 156 internal to patient care device, or an external database 137. A particular configuration database is selected based, at least in part, by patient-specific information such as patient location, age, physical characteristics, or medical characteristics. Medical characteristics include, but are not limited to, patient diagnosis, treatment prescription, medical history, medical records, patient care provider identification, physiological characteristics or psychological characteristics. As used herein, patient-specific information also includes care provider information (e.g., physician identification) or a patient care device's 122 location in the hospital or hospital computer network. Patient care information may be entered through interface device 152, 154, 160 or 162, and may originate from anywhere in network 124, such as, for example, from a pharmacy server, admissions server, laboratory server, and the like.

A memory 156, 158 of the control unit 14 may contain a drug library or libraries, an event log or logs, and pump configuration settings, such as, but not limited to, profiles to be used in particular practice areas such as ICU, PED, etc. The memory may be electronically loadable memory such as non-volatile memory (e.g., EEPROM). Drug libraries stored on pumps (which illustratively contain such information as the drug names, ranges of delivery parameter values such as proper concentrations, dosage units, and dose limits) can be used to perform drug calculation-based infusions in a clinical setting.

A drug library stored within the pump's memory may include clinical order settings such as limits set by the clinical institution for each drug of the library (also termed as "guardrails" herein). Such limits may take the form of maximum and minimum dosages for each drug which may be made dependent on patient factors or other factors associated with delivery of the drug. For example, the dosage limits may vary depending on the weight of the patient or body surface area ("BSA"), depending on the unit or ward of the medical institution in which the drug is being used (for example neonatal care unit (NCU), the intensive care unit (ICU), etc.), and depending on other factors. An alarm may be provided if the nurse sets the pump to operate outside the range between the limits for a particular drug. In some cases, the alarm may be overridden and in other cases it may not. The medical facility may establish "soft" limits for each drug, which may be overridden by the nurse, and "hard" limits which may not. In either case where a limit is exceeded, a pump data log or other processor in communication with the infusion pump may record each such limit event for later analysis where the attempted setting is higher than the maximum or lower than the minimum dosage.

The pump also includes a display for displaying a user interface, including a control panel through which the user can program the programmable controller and a display screen for displaying drug entries from the drug library. Each of the associated sets of drug delivery parameters includes information selected from a group of parameters including drug concentration, drug delivery rate, drug dose, and bolus size. The electronically loaded drug library contains a list of available mode options specifying the units available for expressing drug delivery information, and the drug infusion pump offers the user the list of available mode options from which to make a selection when the electronically loaded drug library is in the pump. In the case of a syringe pump, the electronically loaded drug library may include a list of names of syringe manufacturers identifying syringes that can be used in the drug infusion pump, and the drug infusion pump offers the user the list of names of syringe manufacturers from which to make a selection when the electronically loaded drug library is in the pump. The loaded drug library may include a list of syringe sizes identifying syringes that can be used in the drug infusion pump, and the drug infusion pump offers the user the list of syringe sizes from which to make a selection when the electronically loaded drug library is in said pump. In the case of a peristaltic pump, the electronically loaded drug library may include a list of infusion set manufacturers. A loaded drug library may include a set of features, each of which is either be toggled on or off, and the pump offers the user only the features from among the set of features that are toggled on when the electronically loaded drug library is in said pump.

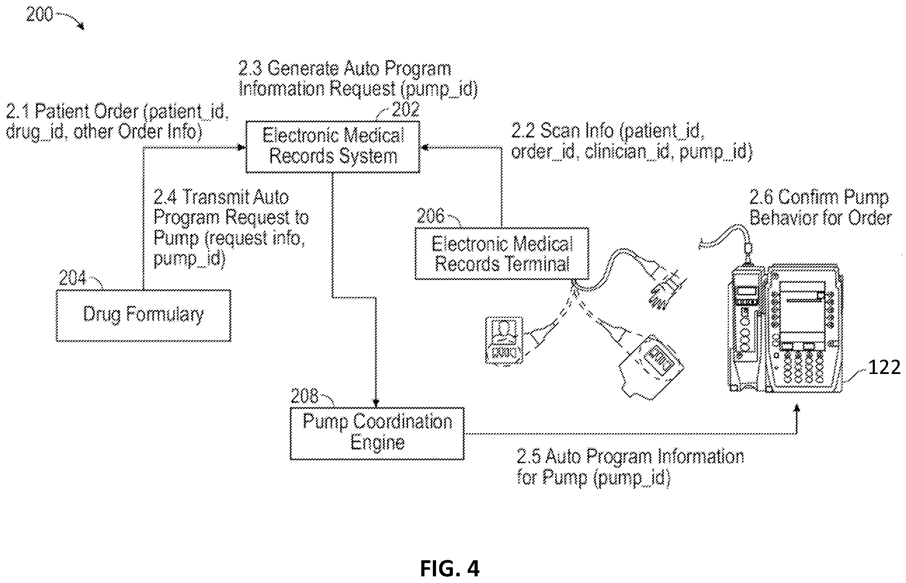

FIG. 4 depicts an example system 200 for automatically programming a medical device, according to aspects of the subject technology. Interoperability between a hospital's electronic medical records (EMR) 202 and medical devices such as infusion device 122 enable pre-population of infusion parameters. Pre-population of infusion parameters may reduce the number of programming screens and key presses required with manual programming of a pump. The implementation of interoperability does not preclude a clinician from manually programming the infusion device. Manual programming may be required in the event of a failure in any component of the interfaced system.

While features may be described with reference to an EMR, the features are applicable to provide auto-programming of medical devices using similar hospital information systems such as pharmacy data management systems (PDMSs). Furthermore, while the features may be described using an infusion pump as the example medical device, the features are applicable to provide auto-programming of other medical devices using barcodes for association such as patient monitors, patient association management systems, or alarms management systems.

A formulary 204 determines which medications can be dispensed within a hospital network. A hospital committee may be formed to determine how medications within that formulary would be applied to the patient care devices 122. While the system of FIG. 4 is described with respect to patient care device 122, the system may be applicable to a standalone infusion device (e.g., syringe pump 30). Configuration definitions (e.g., by hospital unit such as ICU, NICU, Pediatrics, Oncology, Surgery, etc.) are agreed upon and the drugs and typical infusion protocols are established in a medical device drug library ("drug library"). In addition, limitation, or guard rail, conditions are defined in the drug library. When the definitions are complete, then a configuration can be released including the drug library. Pumps at the institution are then updated by transferring the configuration databases into some or all of their pumps. Corresponding updates to the formulary may be shared with other hospital systems such as the pharmacy ordering system or electronic medical records system which may be use formulary information to generate a patient order to deliver a particular drug to a particular patient (e.g., 2.1).

In the clinical field, a clinician may scan a medical item such as an infusate package using a scanner associated with a medical device such as an infusion device 122. For example, a bar code reader (or other data input device) is used to scan the coded drug label, the patient's coded ID band and the caregiver's ID badge, and optionally supplementary prescription information or medical device configuration instructions (including configuration database ID) printed on the label or an accompanying order. The reader/scanner is not required to be integrated with a medical device. The scanner may be part of a separate device such as a medical records terminal 206 (e.g., part of one or more computing devices) connected to the same network 124 as the infusion device 122 and configured with software to function in an overall workflow involving the infusion device 122. The scanning initiates a process by which information pertaining to the item (e.g., scanned from a code affixed to or transmitted by the item) is automatically sent (e.g., 2.2) to the hospital's EMR 202 (e.g., at a centralized server 30) via a network 124. The EMR 202 may confirm the item and generate (e.g., FIG. 2 at 2.3) and send an automated programming request (APR) to the medical device 122 to load parameters pertaining to the item. The parameters may be stored in the medical device 122, but loaded in response to an identifier received from the server. While the examples herein involve an infusion device, any medical device may be configured in the same or similar manner and employ the automated programming error mitigation described herein.

In the depicted implementation, a coordination engine 208 coordinates messages sent from the EMR 202 to the infusion device 122. In the depicted implementation, the EMR 202 transmits an APR (e.g., 2.4) to the pump coordination engine 208 with a device identifier (ID) of the infusion device to receive the APR. The coordination engine then determines whether the infusion device 122 identified by the EMR is available and, if so, forwards the APR to the device 122 (e.g., 2.5). When an APR received by an infusion device 122, the infusion device 122 programs itself according to the parameters of the APR. In some implementations, the APR activates a drug library stored on the device, and the infusion device is programmed according to parameters stored in the drug library for the medication identified in the APR. In some implementations, upon successful programming, the infusion device may automatically initiate operation based on the parameters. In some implementations, the infusion device may confirm the automatically entered parameters (e.g., 2.6). The confirmation may include presenting one or more user interface screens including the parameters and values along with a control element (e.g., a button) that, when activated, causes the infusion device to begin operation based on the parameters. The user interface may include additional or alternative control elements to allow a clinician to adjust an automatically entered parameter based on, for example, professional judgement or changes in patient condition.

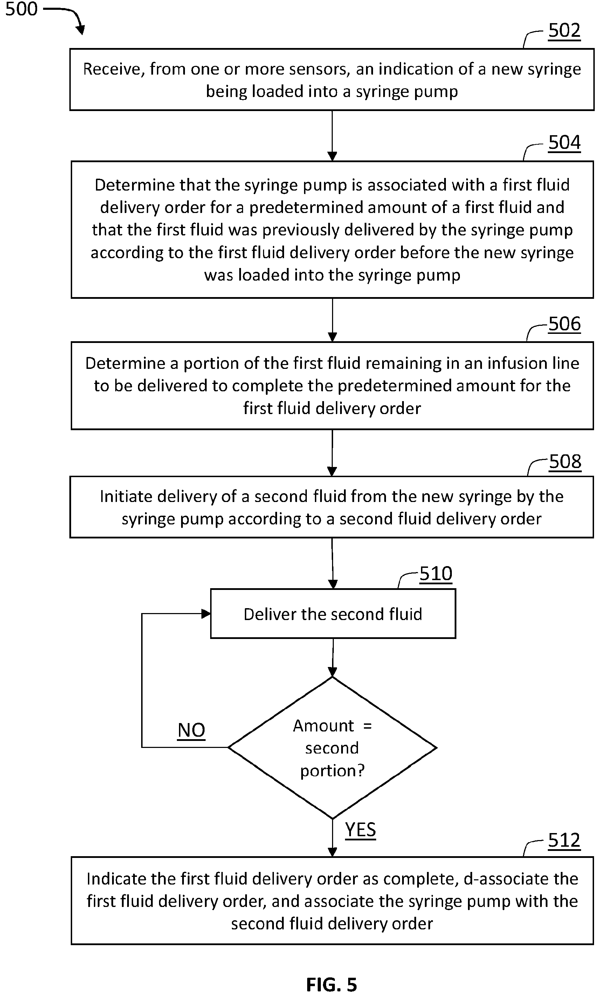

FIG. 5 depicts an example process 500 for accurate delivery of a flush infusion, according to aspects of the subject technology. For explanatory purposes, the various blocks of example process 500 are described herein with reference to FIGS. 1-4, and the components and/or processes described herein. The one or more of the blocks of process 500 may be implemented, for example, by one or more computing devices including, for example, control unit 14 or internal processor of the syringe pump. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 500 are described as occurring in serial, or linearly. However, multiple blocks of example process 500 may occur in parallel. In addition, the blocks of example process 500 need not be performed in the order shown and/or one or more of the blocks of example process 500 need not be performed.

The subject technology, ties multiple infusion delivery orders together in order to ensure delivery of each fluid associated with the orders. For the purpose of this disclosure each "fluid delivery order" means an order for an amount of fluid to be delivered by an infusion device, including any fluid associated with the order that remains in a syringe or that has been pushed out of the delivery syringe and resides in a downstream segment of an IV line 110 (e.g., but not yet delivered to a patient). According to various implementations described herein, a first fluid delivery order for a medication is associated with at least a portion of a second fluid delivery order of a flush type infusate for flushing the remaining amount of medication for the first delivery order out of the IV line 110 to complete the first delivery order. The first delivery order is not identified by the syringe pump as completed until the remaining amount of medication in the IV line is flushed.

While the subject technology is described with regard to a medication and a non-medicinal flush infusate, the subject technology may be configured for two medications. In such implementations, the first medication is reported as complete when enough of the second medication is delivered to push the remaining first medication through the IV line 110.

Prior to (or optionally part of) the depicted process 500, a first syringe is loaded in the syringe pump, and a first fluid is delivered from the first syringe. The first fluid may include, for example, a prescribed medication to be delivered in accordance with a first fluid delivery order. When the syringe is loaded, the sensors associated with the syringe pump (e.g., clamp sensor and/or drive head sensor) detect the loading action and the indication of the syringe being loaded is received by the control unit (e.g., control unit 14 or internal processor of the syringe pump). The syringe pump 30 (operated by the control unit) is caused to deliver the first fluid from the first syringe until the indication of the first syringe being emptied is received. During the infusion, the volume infused is measured and stored in memory. The pump may detect (e.g., by way of one or more sensors) when the syringe becomes empty. When the first syringe becomes emptied, the first fluid delivery order is paused. An empty condition for the first fluid delivery order may be generated. In some implementations, the empty condition and/or volume infused is reported (e.g., via network 124) to an EMR server.

In the depicted example, a new syringe 32 is loaded/received into a receptacle 34 of the syringe pump 30 (while the first fluid delivery order is paused). According to various implementations, the new syringe contains a second fluid, for example, a non-medicinal flush infusate used to flush the remaining first fluid remaining in the IV line 110. The sensors associated with the syringe pump (e.g., clamp sensor and/or drive head sensor) detect the loading action and the indication of the syringe being loaded is received by the control unit (502).

On receiving the indication of the new syringe being loaded, the syringe pump 30 determines that the syringe pump is currently associated with a first fluid delivery order for a predetermined amount of a first fluid, and that at least a first portion of the first fluid was previously delivered by the syringe pump according to the first fluid delivery order before the new syringe was loaded into the syringe pump (504). According to various implementations, the determination (504) is made by the control unit based on electronically stored information associated with the syringe pump 30. For example, data for each infusion (and it's progress) may be stored in an internal memory and/or uploaded to a server (e.g., EMR server). The data may then be accessed/queried when the syringe is loaded or when an infusion is initiated (e.g., by way of user input at the control unit). In some implementations, the determination may be made based on measuring the fluid being delivered. In some implementations, the determination may be made based on receiving an indication that a syringe associated with the first fluid delivery order becoming empty (e.g., based on sensor data) during an infusion of the first fluid.

The control unit determines a second portion of the first fluid remaining to be delivered to complete the predetermined amount of the first fluid for the first fluid delivery order (506). In some implementations, the control unit determines that a first portion of the predetermined amount was previously delivered by the syringe pump according to the first fluid delivery order before the new syringe was loaded into the syringe pump, and the remaining, second portion is determined based on the first portion. In some implementations, the remaining second portion is determined based on receiving a flush command or flush order after delivery of the first fluid, and the second portion remaining is based on an amount designated for the flush.

Delivery of a second fluid from the new syringe is initiated by the syringe pump according to a second fluid delivery order (508). Up until this point, the second portion of the first fluid remaining to be delivered is residing in the IV line 110, but not delivered to the patient. Contemporaneously with initiating the delivery of the second fluid, the syringe pump 30 may generate a restart condition (which may be sent to the EMR server).

The syringe pump is caused to deliver the second fluid (510).

The syringe pump monitors the delivery of the second fluid to determine when the amount of the second fluid satisfies (e.g., is equal to or greater than) the portion of the first fluid remaining to be delivered (e.g., in downstream IV line 110).

When an amount of the second fluid delivered from the new syringe satisfies the second portion of the first fluid remaining to be delivered, the syringe pump automatically, without user intervention, indicates the first fluid delivery order as being complete, de-associates the first fluid delivery order from the syringe pump, and associates the syringe pump with the second fluid delivery order (512). Contemporaneously with associating the syringe pump with the second fluid delivery order, the syringe pump 30 may generate a start condition for the second fluid delivery order (which may be reported to the EMR server). In this manner, the start condition for the second fluid delivery order is not generated until only after the first fluid delivery order is determined to be complete by way of the second remaining portion having been purged from the IV line 110.

In some implementations, indicating the first fluid order as being complete includes associating a type of the second fluid (e.g., a non-medicinal flush infusate) and a flush complete event with the first fluid delivery order. In some implementations, the syringe pump 30 may provide an indication that the second fluid delivery is complete when the new syringe is emptied. In some implementations, the syringe pump 30 may provide an indication that the second fluid delivery is complete when the flush is indicated as complete (e.g., when the first fluid is purged from the IV line, before the new syringe is emptied).

In some implementations, when the amount of the second fluid delivered from the new syringe satisfies the second portion of the first fluid remaining to be delivered, the syringe pump automatically (e.g., without user intervention) prompts a user for a confirmation to continue delivery of the second fluid from the new syringe, and continues delivery of the second fluid from the new syringe responsive to receiving the confirmation from the user.

According to various implementations, the syringe pump 30 is caused to deliver the second fluid by driving a motor of the syringe pump responsive to initiating delivery of the second fluid from the syringe, and continues to delivery of the second fluid from the new syringe by driving the motor of the syringe pump. In this regard, indicating the first fluid delivery order as being complete, de-associating the first fluid delivery order from the syringe pump, associating the syringe pump with the second fluid delivery order, and continuing delivery of the second fluid is performed without stopping the motor.

According to various implementations, the first fluid and second fluid are measured in volumetric units. The syringe pump 30 continuously measures, for the first fluid delivery order, a total volume of the first fluid over a delivery of the first fluid from the first syringe before the empty condition is generated and through the delivery of the second fluid from the new syringe until the amount of the second fluid delivered from the new syringe satisfies the second portion of the first fluid remaining to be delivered. When the total volume is reached (by way of determining that the remaining portion of the first fluid has been purged from the IV line 110), the syringe pump confirms that the measured total volume was delivered according to the first fluid delivery order. The total volume may be displayed by the syringe pump, for example, on display 6, display 114, or other associated display. Unlike legacy pumps, the total volume is confirmed to be delivered (and/or reported to the EMR server) only after enough of the second fluid (from the new syringe) has been infused by the infusion pump to purge the IV line 110.

With brief reference to FIG. 4, the syringe pump 30 may receive, from a remote computing system, an automated programming command to configure the syringe pump to deliver the second fluid (e.g., the non-medicinal flush infusate) according to the second fluid delivery order. Responsive to receiving the automated programming command, without user intervention, the syringe pump may automatically programming the syringe pump to initiate the delivery of the second fluid from the new syringe by the syringe pump according to the second fluid delivery order. In some implementations, the automated programming command may disable a syringe empty alarm currently set to be provided when the first syringe loaded in the syringe pump is emptied.

In some implementations, the automated programming command causes the syringe pump to automatically programming the syringe pump to initiate the delivery of the first fluid from the first syringe by the syringe pump according to the first fluid delivery order and to prompt, when the first syringe is emptied, a user to unload the first syringe and to load the new syringe. When the amount of the second fluid delivered from the new syringe satisfies the second portion of the first fluid remaining to be delivered, the syringe pump 30 may automatically stop delivery of the second fluid.

Many of the above-described example process 600, and related features and applications, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

As used herein, Dose Volume may be synonymous with Diluent Volume. Dose/Diluent Volume can be different than volume to be infused (VTBI). In some implementations, flush (PRN) orders and medication orders are not linked in health information systems (HISs). In some implementations, a flush order and medication order are linked and the linking may be based on, for example, the device providing the order response, patient associated with the order, or time the order response is received at the HIS.

It should be appreciated that the volume infused by a syringe pump refers to a volume the pump has pushed into an administration set (e.g., an intravenous (IV) line downstream of the pump). In this regard, the term "volume infused" as used herein may not be the same as the volume that a patient connected to the pump has received.

Other acronyms or terms used herein:
- eMAR - a patient's electronic medical administration record
- HIS - health information system. The HIS may include the eMAR and partner within the pump interoperable ecosystem. The HIS may include, for example, an EMR server 202 or other server 130 within the healthcare organization.
- KVO - Keep vein open
- Dedicated Medication Line - a clinical technical configuration in which an intravenous line is used exclusively for any prescribed orders for the patient. This line stays connected to the patient and put on standby using a fluid such as normal saline to maintain the set and iv site.

The following example scenarios illustrate features for improving flush administrations according to the aspects described previously with regard FIG. 5. The first four example scenarios generally illustrate how a manual flush can be accurately and safely reported using a syringe pump. The second four scenarios generally illustrate how a flush can be safely and accurately auto-programmed and reported using an automated programming request (APR) as described with regard to FIG. 4. The dosage amounts in the following example scenarios are merely for illustration and may be substituted with "first amount", "second amount", "first portion", "second portion", etc., representative of any predetermined amounts. Control of the pump, as described below, may be performed by one or more processors within the pump, or a control unit or terminal associated with the pump. Reporting, programming and monitoring of the pump may be performed via a user interface on pump or control unit or a terminal associated with the pump (e.g., terminal 132 and/or EMR terminal 206).

### Scenario 1 - Manual Flush, Dose Volume Greater than Available Disposable Volume

In a first example, the disposable IV set 110 is primed with a first amount of a medication (e.g., about 0.5 mL) from a total amount of the fluid in a first fluid delivery order (e.g., 2 mL of medication in the syringe disposable). Is the example, the IV set 110 is primed from the syringe disposable corresponding to the order. Once the IV set 110 is primed, the syringe disposable is loaded into the pump and the remaining medication begins infusing. The infusion results in the medication container running to syringe empty, dose volume remaining to be infused and drug left in the tubing waiting to go to the patient.

As a previously programmed infusion has reached syringe empty with medication remaining in the line, the syringe pump 30 may execute instructions (e.g., firmware, software stored in a non-transitory memory, remote control messages, or the like) or otherwise be configured to perform the following processes to ensure the correct dose is fully delivered and appropriately documented.

In one example, the clinician, using specific flush workflow on the syringe pump 30 (or control unit), programs a volume 0.9 mL as the VTBI and selects START (e.g., activate a control element on a user interface, activate a softkey, activate an electromechanical button) to initiate an infusion according to the programmed parameters. During and/or after programming, the pump may transmit information about the parameters or values therefor to an infusion pump administration system or other HIS.

The pump (or pump module) may then resume the Clindamycin infusion and transmits a (re)start infusion event with a new start code indicating the infusing has restarted but will transition to a flush at a later time. The report may be one or more messages including information about the infusion to the HIS. One example report may be as follows:

| ***Reporting Data*** | **Active Primary Step** | **Pending Flush Step** |
|---|---|---|
| ***Infusion Event:*** | Start | |
| ***Step Type:*** | Intermittent | Flush |
| ***Step State:*** | Infusing | Pending |
| ***State Change Cause:*** | Flush | |
| ***Order ID:*** | MED0101 | NULL |
| ***Infusate Name:*** | Clindamycin | unknown |
| ***Drug Amt.:*** | 200.0000 mg | ---- |
| ***Dil. Vol. (mL):*** | 2.0000 | ---- |
| ***RATE (mL*/*h)*:** | 1.3333 | 1.3333 |
| ***VTBI (mL):*** | 2.0000 | 0.4121 |
| ***Vol. Infused (mL):*** | 1.5121 | |

A. When the volume infused counter reaches 2 mL = dose volume this triggers the pump or control unit to transmit an infusion complete event indicating the dose has been infused. The report may be one or more messages including information about the infusion transmitted to a HIS. One example report may be as follows:

| ***Reporting Data*** | **Active Primary Step** | **Pending Flush Step** |
|---|---|---|
| ***Infusion Event:*** | Complete | |
| ***Step Type:*** | Intermittent | Flush |
| ***Step State:*** | Completed | Pending |
| ***State Change Cause:*** | Flush | |
| ***Order ID:*** | MED0101 | NULL |
| ***Infusate Name:*** | Clindamycin | unknown |
| ***Drug Amt.:*** | 200.0000 mg | ---- |
| ***Dil. Vol. (mL):*** | 2.0000 mL | ---- |
| ***RATE (mL*/*h):*** | 1.3333 | 1.3333 |
| ***VTBI (mL):*** | 2.0000 | 0.4121 |
| ***Vol. Infused (mL):*** | 2.0000 | |

B. The pump or control unit may, immediately following or in close proximity to the complete event and without stopping the motor, transmits a Start event (e.g., to the EMR server 202, 137) for flush indicating the name of the new infusate if known and including an identifier for the active order such as the order ID of medication. The report may be one or more messages including information about the infusion. One example report may be as follows:

| ***Reporting Data*** | **Active Flush Step** |
|---|---|
| ***Infusion Event:*** | Start |
| ***Step Type:*** | Flush |
| ***Step State:*** | Infusing |
| ***State Change Cause:*** | InitStart |
| ***Medication Reference ID:*** | MED0101 |
| ***Flush Order ID:*** | NULL |
| ***Infusate Name:*** | unknown |
| ***Drug Amt.:*** | ---- |
| ***Dil. Vol. (mL):*** | ---- |
| ***RATE (mL*/*h):*** | 1.3333 |
| ***VTBI (mL):*** | 0.4121 |
| ***Vol. Infused (mL):*** | 0.0000 |

When the programmed VTBI of 0.9 mL *(0.4879 of med + 0.4121 of flush)* is reached, the pump or pump module may stop and transmits (directly or via the control unit) a flush complete event indicating the programmed VTBI is complete. The report may be one or more messages including information about the infusion transmitted to a HIS. One example report may be as follows:

| ***Reporting Data*** | **Active Flush Step** |
|---|---|
| ***Infusion Event:*** | Complete |
| ***Step Type:*** | Flush |
| ***Step State:*** | Completed |
| ***State Change Cause:*** | EndProgram |
| ***Medication Reference ID:*** | MED0101 |
| ***Flush Order ID:*** | NULL |
| ***Infusate Name:*** | unknown |
| ***Drug Amt.:*** | ---- |
| ***Dil. Vol. (mL):*** | ---- |
| ***RATE (mL*/*h):*** | 1.3333 |
| ***VTBI (mL):*** | 0.4121 |
| ***Vol. Infused (mL):*** | 0.4121 |

Within the HIS, a clinician reviewing the I/O chart, such as via an eMAR, will see two entries: one entry for 2 mL of Clindamycin and one entry for 0.4121 mL of the infusate named in the flush reports. The flush infusate will be positively and definitively associated with the primary administration of Clindamycin.

### Scenario 2 - Manual Flush, Dose Volume Less Than Available Disposable Volume

In another example, the disposable tubing is pre-primed with 0.5 mL of a solution. The syringe disposable is loaded into the pump and the medication begins infusing. The medication infusion results in the medication container running to infusion complete, all the dose volume was infused but there is medication left in the tubing waiting to go to the patient.

As a previously programmed infusion has reached infusion complete with medication remaining in the line, the syringe pump may execute instructions (e.g., firmware, software stored in a non-transitory memory, remote control messages, or the like) or otherwise be configured to perform the following processes to ensure the correct dose is fully delivered and appropriately documented.

In this example, the clinician, using specific flush workflow on the device, programs a volume 0.9 mL as the VTBI and selects START (e.g., activate a control element on a user interface, activate a softkey, activate an electromechanical button) to initiate an infusion according to the programmed parameters. During and/or after programming, the pump may transmit information about the parameters or values therefor to an infusion pump administration system or other HIS.

The pump (or pump module) may then start infusing and transmit (via the pump or control unit) a Start event for flush indicating the name of the new infusate if known and include an identifier for the active order such as the order ID of medication. The report may be one or more messages including information about the infusion transmitted to a HIS. One example report may be as follows:

| ***Reporting Data*** | **Active Flush Step** |
|---|---|
| ***Infusion Event:*** | Start |
| ***Step Type:*** | Flush |
| ***Step State:*** | Infusing |
| ***State Change Cause:*** | InitStart |
| ***Medication Reference ID:*** | Clindamycin + Pump ID |
| ***Flush Order ID:*** | NULL |
| ***Infusate Name:*** | unknown |
| ***Drug Amt.:*** | ---- |
| ***Dil. Vol. (mL):*** | ---- |
| ***RATE (mL*/*h):*** | 1.3333 |
| ***VTBI (mL):*** | 0.9000 |
| ***Vol. Infused (mL):*** | 0.0000 |

When the VTBI of 0.9 mL is reached, the pump or pump module may stop infusing and transmit (directly or via the control unit) a flush complete event indicating the VTBI as programmed is complete. The report may be one or more messages including information about the infusion transmitted to a HIS. One example report may be as follows:

| ***Reporting Data*** | **Active Flush Step** |
|---|---|
| ***Infusion Event:*** | Complete |
| ***Step Type:*** | Flush |
| ***Step State:*** | Completed |
| ***State Change Cause:*** | EndProgram |
| ***Medication Reference ID:*** | Clindamycin + Pump ID |
| ***Flush Order ID:*** | NULL |
| ***Infusate Name:*** | unknown |
| ***Drug Amt.:*** | ---- |
| ***Dil. Vol. (mL):*** | ---- |
| ***RATE (mL*/*h):*** | 1.3333 |
| ***VTBI (mL):*** | 0.9000 |
| ***Vol. Infused (mL):*** | 0.9000 |

Within the HIS, a clinician reviewing the I/O chart, such as via an eMAR, will see two entries: one entry for 2 mL of Clindamycin and one entry for 0.9 mL of the infusate named in the flush reports. The flush infusate will be positively and definitively associated with the primary administration of Clindamycin.

### Scenario 3 - Manual Flush for Micro Volume Infusion

In one example, the syringe pump 30 primes disposable tubing 110 with small volume medication (dose volume less than tubing volume) and requires immediate start of a flush disposable. The flush results in disposable running to infusion complete, all dose volume infused and no medication left in tubing.
1. After manually pushing the medication into the IV line, the clinician loads the new flush disposable (syringe) into the syringe pump 30.
2. The syringe pump 30 prompts the clinician to choose the medication from the drug library, program the infusion parameters:
3. The syringe pump 30 (or control unit 14) may proceed to a primary infusion setup screen and display the following setup parameters:

| | |
|---|---|
| a. | Drug Title = Clindamycin |
| b. | Rate = 1.333 mL/h |
| c. | VTBI = 2 mL |
| d. | Avail. Vol. = 3.015 mL |

4. In this example, the clinician reviews the parameters and selects START (e.g., activate a control element on a user interface, activate a softkey, activate an electromechanical button) to initiate an infusion according to the programmed parameters.
5. The pump (or pump module) starts infusing and the infusion pump 30 (e.g., module of FIG. 1B or standalone of FIG. 2) or control unit associated with the pump, may report multiple infusion events for the program (e.g., via the display or to the server 202, 137).
6. Once infusion is complete on the pump and within the HIS, a clinician reviewing the I/O chart, such as via an eMAR, will see an entry for 2 mL of Clindamycin in the patient's chart.
7. In this example, the clinician, using specific flush workflow on the syringe pump or control unit, programs additional volume 1 mL as the VTBI and selects START (e.g., activate a control element on a user interface, activate a softkey, activate an electromechanical button) to initiate an infusion according to the programmed parameters. During and/or after programming, the pump may transmit information about the parameters or values therefor to an infusion pump administration system or other HIS (e.g., to EMR server 202 or server 137).
8. The pump 30 (or pump module) starts infusing and the pump or control unit or server/client terminal associated with the pump module, may report a start event of the flush order (e.g., via a display and/or to a server). The report may include a reference to the previous medication such as an order ID, drug name and conc pair and the flush solution, if known. The report may be one or more messages including information about the infusion to the HIS. One example report may be as follows:

| Reporting Data | **Active Flush Step** |
|---|---|
| ***Infusion Event:*** | Start |
| ***Step Type:*** | Flush |
| ***Step State:*** | Infusing |
| ***State Change Cause:*** | InitStart |
| ***Medication Reference ID:*** | Clinda3 |
| ***Flush Order ID:*** | Normal Saline |
| ***Infusate Name:*** | unknown |
| ***Drug Amt.:*** | ---- |
| ***Dil. Vol. (mL):*** | ---- |
| ***RATE (mL*/*h):*** | 1.3333 |
| ***VTBI (mL):*** | 1.0000 |
| ***Vol. Infused (mL):*** | 0.0000 |

9. When the VTBI of 1 mL is reached, the pump 30 may stop and report (on a display or to a server) a flush complete event. The report may be one or more messages including information about the infusion transmitted to a HIS. One example report may be as follows:

| ***Reporting Data*** | **Active Flush Step** |
|---|---|
| ***Infusion Event:*** | Complete |
| ***Step Type:*** | Flush |
| ***Step State:*** | Completed |
| ***State Change Cause:*** | EndProgram |
| ***Medication Reference ID:*** | Clinda3 |
| ***Flush Order ID:*** | Normal Saline |
| ***Infusate Name:*** | unknown |
| ***Drug Amt.:*** | ---- |
| ***Dil. Vol. (mL):*** | ---- |
| ***RATE (mL*/*h):*** | 1.3333 |
| ***VTBI (mL):*** | 1.0000 |
| ***Vol. Infused (mL):*** | 1.0000 |

10. Within the HIS, a clinician reviewing the I/O chart, such as via an eMAR, may see two entries: one entry for 2 mL of Clindamycin and one entry for 1 mL of the infusate named in the flush reports. The flush infusate may be positively and definitively associated with the primary administration of Clindamycin.

### Scenario 4 - Manual Flush, First To Push A Primed Volume

In this example, a dedicated medication line is currently in standby connected to the patient. A time critical order of Vancomycin may be identified for delivery to the patient. A peak lab draw may be ordered for this dose. The clinician may load the medication disposable and push the Vancomycin to the bottom of the tubing to avoid any delay in administration and understand the exact time the drug administration will begin.

Three preconditions may exist before executing the workflow.
- The pump is loaded with a dedicated medication line 110 with a normal saline disposable, but is set to standby for the next medication order.
- The tubing volume between pump and patient is 1.1 mL
- The flow rate of the order is 1.25 mL/h

It may also be desirable to conclude the workflow in a known state. In this example, the post-condition, after the workflow, is that the dedicated medication line is loaded with normal saline disposable and returned to standby for a subsequent Medication Reference.
1. The clinician replaces the normal saline disposable with the medication disposable in the pump or pump module.
2. Clinician selects Vancomycin in 2.5 mL from the drug library on the device and proceeds with programming.
3. The pump 30 (or control unit) may proceed to a primary infusion setup screen and display the following setup parameters:

| | |
|---|---|
| a. | Drug Title = Vancomycin |
| b. | Rate = 1.25 mL/h |
| c. | VTBI = 2.5 mL |
| d. | Duration = 2 hours 0 minutes |
| e. | Avail. Vol. = 2.49 mL |

4. In this example, the clinician reviews the parameters and selects PRIME (e.g., activate a control element on a user interface, activate a softkey, activate an electromechanical button) to initiate priming the Vancomycin disposable using the software prime feature into the tubing and Vancomycin medication towards the patient.
5. The pump (or pump module) starts priming at 250 mL/h and the pump or control unit or server/client terminal associated with the pump module and report a prime start event to the HIS. The report may be one or more messages including information about the priming to the HIS. One example report may be as follows:

| ***Reporting Data*** | **Active Prime Step** |
|---|---|
| ***Infusion Event:*** | Start |
| ***Step Type:*** | Prime |
| ***Step State:*** | Infusing |
| ***State Change Cause:*** | InitStart |
| ***Medication Reference ID:*** | Clinda4 |
| ***Flush Order ID:*** | NULL |
| ***Infusate Name:*** | Vancomycin |
| ***Drug Amt.:*** | 25.0000 |
| ***Dil. Vol. (mL):*** | 2.5000 |
| ***RATE (mL*/*h):*** | 250.0000 |
| ***VTBI (mL):*** | ---- |
| ***Vol. Primed (mL):*** | 0.0000 |

6. The clinician may stop the priming once the Vancomycin has reached the patient. Stopping the priming may include activating a control element of the pump or associated control unit to transmit a control signal to stop the priming. In some implementations, the clinician may deactivate (e.g., stop pressing, depress) a control element to control the pump to stop priming.
7. The pump, pump module, or control unit associated with the pump, may indicate (e.g., display) 1.1 mL volume primed and report a prime stop event to the HIS. The report may be one or more messages including information about the priming to the HIS. One example report may be as follows:

| ***Reporting Data*** | **Active Prime Step** |
|---|---|
| ***Infusion Event:*** | Stop |
| ***Step Type:*** | Prime |
| ***Step State:*** | Completed |
| ***State Change Cause:*** | EndProgram |
| ***Medication Reference ID:*** | Clinda4 |
| ***Flush Order ID:*** | NULL |
| ***Infusate Name:*** | Vancomycin |
| ***Drug Amt.:*** | 25.0000 |
| ***Dil. Vol. (mL):*** | 2.5000 |
| ***RATE (mL*/*h):*** | 250.0000 |
| ***VTBI (mL):*** | ---- |
| ***Vol. Primed (mL):*** | 1.1001 |

8. In this example, the clinician may exit the prime feature and activate or return to an infusion programming interface.
9. The pump or control unit may proceed to a primary infusion step setup screen and display the following setup parameters:

| | |
|---|---|
| f. | Drug Title = Vancomycin |
| g. | Rate = 1.25 mL/h |
| h. | VTBI = 2.5 mL |
| i. | Duration = 2 hours 0 minutes |
| J. | Avail. Vol. = 1.39 mL |

10. In this example, the clinician reviews the parameters and selects START (e.g., activate a control element on a user interface, activate a softkey, activate an electromechanical button) to initiate the primary infusion according to the setup parameters.
11. The pump (or pump module) starts infusing Vancomycin and the pump or control unit or server/client terminal associated with the pump module, may reports a start event. The report may be one or more messages including information about the infusion to the HIS. One example report may be as follows:

| ***Reporting Data*** | **Active Primary Step** |
|---|---|
| ***Infusion Event:*** | Start |
| ***Step Type:*** | Intermittent |
| ***Step State:*** | Infusing |
| ***State Change Cause:*** | InitStart |
| ***Order ID:*** | NULL |
| ***Infusate Name:*** | Vancomycin |
| ***Drug Amt.:*** | 25.0000 |
| ***Dil. Vol. (mL):*** | 2.5000 |
| ***RATE (mL*/*h):*** | 1.2500 |
| ***VTBI (mL):*** | 2.5000 |
| ***Vol. Infused (mL):*** | 0.0000 |

12. Within the HIS, a clinician reviewing an entry item reporting 0 mL infused of Vancomycin in, for example, the patient's eMAR. The clinician may be prompted to document 1.1 mL of Vancomycin prime volume.
13. Via the HIS (e.g., eMAR), the clinician can associate the 1.1 mL of volume to normal saline for the patient. In some implementations, the association may be suggested or identified by the HIS based on the incoming infusion event reports and other information in the patient's electronic medical record.
14. After 1 hour and 15 minutes, the pump or pump module may report (e.g., alarm) empty disposable, stop, and report (directly or via the control unit) an infusion stop event. The report may be one or more messages including information about the infusion transmitted to a HIS. One example report may be as follows:

| ***Reporting Data*** | **Active Primary Step** |
|---|---|
| ***Infusion Event:*** | Stop |
| ***Step Type:*** | Intermittent |
| ***Step State:*** | Alarming |
| ***State Change Cause:*** | Alarm Stop |
| ***Order ID:*** | NULL |
| ***Infusate Name:*** | Vancomycin |
| ***Drug Amt.:*** | 25.0000 |
| ***Dil. Vol. (mL):*** | 2.5000 |
| ***RATE (mL*/*h):*** | 1.2500 |
| ***VTBI (mL):*** | 2.5000 |
| ***Vol. Infused (mL):*** | 1.3944 |

15. The workflow of example Scenario 1 may be executed to complete the dose and return the medication line back to a standby state.

### Scenario 4 - Alternative

In one example, the following steps may be altered:
4. Clinician selects flush workflow from the device in the drug library on the device and proceeds with programming.
5. The pump or control unit displays the primary infusion step setup screen as follows:

| | |
|---|---|
| a. | Title Bar = Prime-Flush |
| b. | Rate = _mL/h |
| c. | VTBI = _mL |
| d. | Avail. Vol. = 2.49 mL |

6. The clinician programs 250 mL/h and VTBI of 1.1 mL and starts an infusion.
7. The syringe pump 30 starts infusing and the pump or control unit reports an infusion start event as follows including a unique ID to be included with the medication report:

| ***Reporting Data*** | **Active Primary Step** |
|---|---|
| ***Infusion Event:*** | Start |
| ***Step Type:*** | Fluid |
| ***Step State:*** | Infusing |
| ***State Change Cause:*** | InitStart |
| ***Order ID:*** | flush 19 |
| ***Infusate Name:*** | Prime-Flush |
| ***RATE (mL*/*h):*** | 250.0000 |
| ***VTBI (mL):*** | 1.1000 |
| ***Vol. Infused (mL):*** | 0.0000 |

8. The module stops infusing, switches to KVO, and the pump or control unit reports a prime stop event as follows:

| ***Reporting Data*** | **Active Prime Step** |
|---|---|
| ***Infusion Event:*** | Stop |
| ***Step Type:*** | Prime |
| ***Step State:*** | Completed |
| ***State Change Cause:*** | EndProgram |
| ***Order ID:*** | flush19 |
| ***Infusate Name:*** | Prime-Flush |
| ***RATE (mL*/*h):*** | 250.0000 |
| ***VTBI (mL):*** | 1.1000 |
| ***Vol. Infused (mL):*** | 1.1000 |

9. Clinician ends KVO step and returns in 30 mins to start manually start Vancomycin infusion.

### Scenario 5 - Flush APR, Dose Volume Greater Than The Available Disposable Volume

In some implementations, a syringe pump 30 may be configured to deliver a primary fluid delivery order and a flush order using automated programming requests (APRs). In one example, the IV line 110 is primed with about 0.5 mL of medication from the 2mL of the syringe disposable. The syringe disposable is loaded into the pump and the remaining medication begins infusing. The infusion results in medication container running to syringe empty, dose volume remaining to be infused and drug left in the tubing waiting to go to the patient.

In this example, three preconditions may exist before executing the workflow.
- A PRN flush order exists in the HIS
- The Dose Volume is greater than the volume in the disposable
- A previously programmed infusion is nearing syringe empty

1. Unlike in example Scenario 1, rather than selecting and programming a flush, a clinician may send (directly or indirectly) a FLUSH APR PRN-order for Normal Saline to the pump (e.g., using EMR terminal 206 of FIG. 4). An example APR may be as follows:

| **Flush APR Details** | |
|---|---|
| ***Clinician ID:*** | **RatchedRN** |
| ***Patient ID:*** | W.Yueh |
| ***Order Type:*** | PrnFlush |
| ***Flush Order ID:*** | FLUSHIE2000 |
| ***Flush Solution:*** | Normal Saline |
| ***VTBI:*** | 1.0000 |

In some implementations, the flush APR may include an "additional volume" parameter in place of, or in addition to, the VTBI parameter. The additional volume parameter would indicate how much additional volume of fluid to push into the administration line 110. This may help more accurately reflect the volume actually delivered to the patient.
2. The pump or associated control unit may validate the order and, upon successful validation, accept the APR request as a Flush Request.
3. The pump or associated control unit may silence any syringe empty alarms and prompt to load the flush disposable. This will stop the infusion if it has not already stopped due to the syringe empty or completed volume infused.
4. In this example, the clinician unloads the medication syringe and connects and loads the flush syringe containing about 2 mL of Normal Saline and proceeds with programming.
5. The pump or control unit may proceed to a flush step setup screen and display the following setup parameters:

| | |
|---|---|
| a. | Drug Title = Clindamycin² - Flush¹ |
| b. | Flush Name = Normal Saline¹ |
| c. | Rate = 1.333² mL/hr |
| d. | VTBI = 1¹ mL |
| e. | Avail. Vol. = 1.956² mL |

| | |
|---|---|
| Notes: *1. Populated from APR data* *2. Populated from Pump data* | |

6. In this example, the clinician reviews program and selects START (e.g., activate a control element on a user interface (e.g., of the pump, terminal 132, 206), activate a softkey, activate an electromechanical button) to initiate a flush according to the programmed parameters. During and/or after programming, the pump may transmit information about the parameters or values therefor to an infusion pump administration system or other HIS.
7.
A. The pump 30 may then resume the infusion and transmit a report (via the pump or control unit) to the HIS. The report may be one or more messages including information about the infusion transmitted to a HIS. One example report may be as follows:

| ***Reporting Data*** | **Active Primary Step** | **Pending Flush Step** |
|---|---|---|
| ***Infusion Event:*** | Start | |
| ***Step Type:*** | Intermittent | Flush |
| ***Step State:*** | Infusing | Pending |
| ***State Change Cause:*** | Flush | |
| ***Order ID:*** | MED0101 | FLUSHIE2000 |
| ***Infusate Name:*** | Clindamycin | Normal Saline |
| ***Drug Amt.:*** | 200.0000 mg | ---- |
| ***Dil. Vol. (mL):*** | 2.0000 | ---- |
| ***RATE (mL*/*h):*** | 1.3333 | 1.3333 |
| ***VTBI (mL):*** | 2.0000 | 0.5121 |
| ***Vol. Infused (mL):*** | 1.5121 | |

| | | |
|---|---|---|
| Note that the programmed VTBI was 1.0 mL but reported here as 0.5121 mL. This is because the system will account for the amount of flush actually needed for the associated primary infusion. In this example, the reported FLUSH VTBI = Programmed VTBI - remaining dose volume. (i.e., for this example, 1mL - .4879mL = .5121mL). | | |

B. The pump or control unit may, immediately preceding, following, or in close proximity to the start event, transmit a flush "Program Confirmation event" to the HIS (e.g., server 130, 202) to acknowledge that the APR was confirmed and is pending on the module. In some implementations, the report may be included as part of the start event report. The report may be one or more messages including information about the infusion. One example report may be as follows:

| ***Reporting Data*** | **Flush Step** |
|---|---|
| ***Program Event:*** | Program Confirmation |
| ***Medication Reference ID:*** | MED0101 |
| ***Flush Order ID:*** | FLUSHIE2000 |
| ***Infusate Name:*** | Normal Saline |
| ***Drug Amt.:*** | ---- |
| ***Dil. Vol. (mL):*** | ---- |
| ***RATE (mL*/*h):*** | 1.3333 |
| ***Programmed VTBI (mL):*** | 1.0000 |
| ***Flush VTBI (mL):*** | 0.5121 |

8.
A. When the volume infused counter reaches 2 mL = dose volume, the pump or control unit or server/client terminal associated with the pump module may transmit an infusion complete event to the HIS. The report may be one or more messages including information about the infusion transmitted to a HIS. One example report may be as follows:

| ***Reporting Data*** | **Active Primary Step** | **Pending Flush Step** |
|---|---|---|
| ***Infusion Event:*** | Complete | |
| ***Step Type:*** | Intermittent | Flush |
| ***Step State:*** | Completed | Pending |
| ***State Change Cause:*** | Flush | |
| ***Order ID:*** | MED0101 | FLUSHIE2000 |
| ***Infusate Name:*** | Clindamycin | Normal Saline |
| ***Drug Amt.:*** | 200.0000 mg | ---- |
| ***Dil. Vol. (mL):*** | 2.0000 mL | ---- |
| ***RATE (mL*/*h):*** | 1.3333 | 1.3333 |
| ***VTBI (mL):*** | 2.0000 | 0.5121 |
| ***Vol. Infused (mL):*** | 2.0000 | |

The step state may be represented using a different state such as switch containers. The selection of step state may be based on the original APR or subsequent parameters received at the pump or pump module or control unit.

B. The infusion complete report may be closely followed in time by transmission of a start event for flush with order ID of medication. The report may be one or more messages including information about the infusion to the HIS. One example report may be follows:

| ***Reporting Data*** | **Active Flush Step** |
|---|---|
| ***Infusion Event:*** | Start |
| ***Step Type:*** | Flush |
| ***Step State:*** | Infusing |
| ***State Change Cause:*** | InitStart |
| ***Medication Reference ID:*** | MED0101 |
| ***Flush Order ID:*** | FLUSHIE2000 |
| ***Infusate Name:*** | Normal Saline |
| ***Drug Amt.:*** | --- |
| ***Dil. Vol. (mL):*** | --- |
| ***RATE (mL*/*h):*** | 1.3333 |
| ***VTBI (mL):*** | 0.5121 |
| ***Vol. Infused (mL):*** | 0.0000 |

The pump may stop and report (directly or via the control unit) a flush complete event. The report may be one or more messages including information about the infusion transmitted to a HIS. One example report may be as follows:

| ***Reporting Data*** | **Active Flush Step** |
|---|---|
| ***Infusion Event:*** | Complete |
| ***Step Type:*** | Flush |
| ***Step State:*** | Completed |
| ***State Change Cause:*** | EndProgram |
| ***Medication Reference ID:*** | MED0101 |
| ***Flush Order ID:*** | FLUSHIE2000 |
| ***Infusate Name:*** | Normal Saline |
| ***Drug Amt.:*** | --- |
| ***Dil. Vol. (mL):*** | --- |
| ***RATE (mL*/*h):*** | 1.3333 |
| ***VTBI (mL):*** | 0.5121 |
| ***Vol. Infused (mL):*** | 0.5121 |

10. Within the HIS, a clinician reviewing the I/O chart, such as via an eMAR, will see entries for 2 mL of Clindamycin and 0.5121 mL of the infusate named in the flush reports. The flush infusate will be positively and definitively associated with the primary administration of Clindamycin.

### Scenario 6 - Flush APR, Dose Volume Less Than Available Disposable Volume

In some implementations, the pump may be configured to deliver a primary delivery order and flush order using APRs. A disposable tubing may be pre-primed with, for example, 0.5 mL of a solution from a syringe. The syringe may be loaded into the pump and the medication begins infusing. The medication step results in medication container running to infusion complete, all the dose volume was infused by pump but there is medication volume left in the tubing waiting to go to the patient.

In this example, three preconditions may exist before executing the workflow.
- A PRN flush order exists in the HIS.
- The Dose Volume is less than the volume in the disposable.
- A previously programmed infusion has just completed infusing all the programmed VTBI.

1. Unlike in example Scenario 2, rather than selecting and programming a flush, a clinician may send (directly or indirectly) a FLUSH APR order for diluted Heparin to the pump (as a flush request). An example APR may be as follows:

| **Flush APR Details** | |
|---|---|
| *Clinician ID:* | RatchedRN |
| *Patient ID:* | W.Yueh |
| *Order Type:* | PrnFlush |
| *Order ID:* | FLUSHIE2000 |
| *Flush Solution:* | Normal Saline |
| *VTBI:* | 0.5000 |

2. In some implementations, the flush APR may include an "additional volume" parameter in place of, or in addition to, the VTBI parameter. The additional volume parameter would indicate how much additional volume of fluid to push into the administration line. This may help more accurately reflect the volume actually delivered to the patient. The pump or associated control unit may validate the order and, upon successful validation, accept the APR request as a flush request.
3. The pump or associated control unit may silence any alarms related to the stopped infusion and prompt to load the flush disposable. This will stop the infusion if it has not already stopped due to the syringe empty or completed volume infused.
4. The pump or associated control unit may alert a user that Heparin was scanned as a flush solution based on a configuration. In addition, the pump may alert the user that there could be a drug interaction between the flush solution and medication. Finally, the pump may prompt a user to load flush disposable
5. In this example, the clinician unloads the medication syringe and connects and loads the flush syringe containing about 2 mL of diluted Heparin.
6. The pump or associated control unit may proceed to a flush step setup screen and display the following setup parameters:

| | |
|---|---|
| a. | Title Bar = Clindamycin² - Flush w/ Diluted Heparin¹ |
| b. | Rate = 1.333² mL/hr |
| c. | VTBI = 0.5¹ mL |
| d. | Avail. Vol. = 2.091² mL |

| | |
|---|---|
| Notes: *1. Populated from APR data* *2. Populated from Pump data* | |

7. In this example, the clinician reviews program and selects START (e.g., activate a control element on a user interface, activate a softkey, activate an electromechanical button) to initiate a flush according to the programmed parameters. During and/or after programming, the pump may transmit information about the parameters or values therefor to an infusion pump administration system or other HIS.
8. The pump 30 may then begin infusing and transmit a report (via the pump or control unit) a flush start event to the HIS (e.g., server 130, 202). The report may be one or more messages including information about the infusion transmitted to a HIS. One example report may be as follows:

| ***Reporting Data*** | **Active Flush Step** |
|---|---|
| ***Infusion Event:*** | Start |
| ***Step Type:*** | Flush |
| ***Step State:*** | Infusing |
| ***State Change Cause:*** | InitStart |
| ***Medication Reference ID:*** | 5121064 |
| ***Flush Order ID:*** | FLUSHIE2000 |
| ***Infusate Name:*** | Diluted Heparin |
| ***Drug Amt.:*** | --- |
| ***Dil. Vol. (mL):*** | --- |
| ***RATE (mL*/*h):*** | 1.3333 |
| ***VTBI (mL):*** | 0.5000 |
| ***Vol. Infused (mL):*** | 0.0000 |

9. When the VTBI of 0.5 mL from the APR is reached, the pump may stop and report (directly or via the control unit) a flush complete event. The report may be one or more messages including information about the infusion transmitted to a HIS. One example report may be as follows:

| ***Reporting Data*** | **Active Flush Step** |
|---|---|
| ***Infusion Event:*** | Complete |
| ***Step Type:*** | Flush |
| ***Step State:*** | Completed |
| ***State Change Cause:*** | EndProgram |
| ***Medication Reference ID:*** | 5121064 |
| ***Flush Order ID:*** | FLUSHIE2000 |
| ***Infusate Name:*** | Diluted Heparin |
| ***Drug Amt.:*** | ---- |
| ***Dil. Vol. (mL):*** | ---- |
| ***RATE (mL*/*h):*** | 1.3333 |
| ***VTBI (mL):*** | 0.5000 |
| ***Vol. Infused (mL):*** | 0.5000 |

10. Within the HIS (e.g., via server 130, terminal 132, 206, or on a pump display 6, 80, 114), a clinician reviewing the I/O chart, such as via an eMAR, will see entries for 2 mL of Clindamycin and 0.5 mL of Diluted Heparin. The flush infusate will be positively and definitively associated with the primary administration of Clindamycin.

### Scenario 7 - Flush APR For Micro Volume Infusion

In some implementations, the syringe pump 30 may be configured to deliver a primary fluid delivery order and flush order using APRs. In one example, the IV line 110 is primed with small volume medication (dose volume less than tubing volume) and a flush is initiated from a flush disposable. The flush results in the disposable running to infusion complete, all dose volume infused, and no medication (e.g., from the previous order) left in the IV tubing.

In this example, preconditions may exist before executing the workflow.
- The Dose Volume is less than the tubing volume
- Dose volume is manually pushed into tubing between pump and patient

1. After manually pushing the medication into the IV line and unlike example Scenario 3, the clinician scans (e.g., barcode or other wireless identifier) empty med disposable, patient, and pump. The clinician may use, for example, a barcode scanner associated with the EMR terminal 206, as described with regard to FIG. 4.
2. The clinician confirms medication order in HIS and sends medication APR to pump.
3. Clinician discards the empty medication disposable and loads Flush disposable into the pump or pump module.
4. The pump or control unit may proceed to a primary infusion setup screen and display the following setup parameters:

| | |
|---|---|
| a. | Drug Title = Clindamycin |
| b. | Rate = 1.333 mL/h |
| c. | VTBI = 2 mL |
| d. | Avail. Vol. = 3.015 mL |

5. In this example, the clinician reviews the parameters and selects START (e.g., activate a control element on a user interface of the display 6, 80, 114, activate a softkey, activate an electromechanical button on the pump or control unit) to initiate an infusion according to the programmed parameters.
6. The pump 30 starts infusing and the pump or control unit or server/client terminal associated with the pump module, may report a start event. The report may be one or more messages including information about the infusion to the HIS. One example report may be as follows:

| ***Reporting Data*** | **Active Primary Step** |
|---|---|
| ***Infusion Event:*** | Start |
| ***Step Type:*** | Intermittent |
| ***Step State:*** | Infusing |
| ***State Change Cause:*** | InitStart |
| ***Order ID:*** | 1984BYGO |
| ***Infusate Name:*** | Clindamycin |
| ***Drug Amt.:*** | 200.0000 mg |
| ***Dil. Vol. (mL):*** | 2.0000 |
| ***RATE (mL*/*h):*** | 1.3333 |
| ***VTBI (mL):*** | 2.0000 |
| ***Vol. Infused (mL):*** | 0.0000 |

7. When the programmed VTBI of 2 mL is reached, the pump may stop and report (directly or via the control unit or terminal) an infusion complete event. The report may be one or more messages including information about the infusion transmitted to a HIS. One example report may be as follows:

| ***Reporting Data*** | **Active Primary Step** |
|---|---|
| ***Infusion Event:*** | Complete |
| ***Step Type:*** | Intermittent |
| ***Step State:*** | Completed |
| ***State Change Cause:*** | EndProgram |
| ***Order ID:*** | 1984BYGO |
| ***Infusate Name:*** | Clindamycin |
| ***Drug Amt.:*** | 200.0000 mg |
| ***Dil. Vol. (mL):*** | 2.0000 |
| ***RATE (mL*/*h):*** | 1.3333 |
| ***VTBI (mL):*** | 2.0000 |
| ***Vol. Infused (mL):*** | 2.0000 |

8. Within the HIS, a clinician reviewing the I/O chart, such as via an eMAR, will see an entry for 2 mL of Clindamycin in patient's chart.
9. To provide flush instructions, a clinician may send (directly or indirectly via the terminal) a FLUSH APR order for Normal Saline to the pump. An example APR may be as follows:

| **Flush APR Details** | |
|---|---|
| *Clinician ID:* | RatchedRN |
| *Patient ID:* | W.Yueh |
| *Order Type:* | PrnFlush |
| *Order ID:* | FLUSHIE2000 |
| *Flush Solution:* | Normal Saline |
| *VTBI:* | 0.5000 |

In some implementations, the Flush APR may include an "additional volume" parameter in place of, or in addition to, the VTBI parameter. The additional volume parameter would indicate how much additional volume of fluid to push into the administration line. This may help more accurately reflect the volume actually delivered to the patient.
10. The pump or associated control unit may validate the order and, upon successful validation, accept the APR request as a Flush Request.
11. The pump or associated control unit may prompt a user to load flush disposable
12. Because the flush disposable is already loaded, the clinician may simulate an unload and reload for the flush disposable. In some implementations, the pump or control unit or terminal associated with the pump may offer a load override option. The override option may be dynamically presented such as based on primary medication, volume to be infused, etc.
13. The pump or control unit or associated terminal proceeds to the flush step setup screen. An example set of parameters and values displayed are the following:

| | |
|---|---|
| a. | Drug Title = Clindamycin - Flush |
| b. | Rate = 1.333 mL/h |
| c. | VTBI = 0.5 mL |
| d. | Avail. Vol. = 1.015 mL |

14. In this example, the clinician may review the parameters and selects START (e.g., activate a control element on a user interface, activate a softkey, activate an electromechanical button) to initiate an infusion according to the programmed parameters.
15. The pump 30 starts infusing and the pump or control unit or server/client terminal associated with the pump module, may reports a start event. The report may include the Order ID of Medication, if known. The report may be one or more messages including information about the infusion to the HIS. One example report may be as follows:

| ***Reporting Data*** | **Active Flush Step** |
|---|---|
| ***Infusion Event:*** | Start |
| ***Step Type:*** | Flush |
| ***Step State:*** | Infusing |
| ***State Change Cause:*** | InitStart |
| ***Medication Reference ID:*** | 1984BYGO |
| ***Flush Order ID:*** | FLUSHIE2000 |
| ***Infusate Name:*** | Normal Saline |
| ***Drug Amt.:*** | ---- |
| ***Dil. Vol. (mL):*** | ---- |
| ***RATE (mL*/*h):*** | 1.3333 |
| ***VTBI (mL):*** | 0.5000 |
| ***Vol. Infused (mL):*** | 0.0000 |

16. When the VTBI of 1 mL is reached, the pump or pump module may stop and report (directly or control unit or terminal associated with the pump) a flush complete event. The report may be one or more messages including information about the infusion transmitted to a HIS. One example report may be as follows:

| ***Reporting Data*** | **Active Flush Step** |
|---|---|
| ***Infusion Event:*** | Complete |
| ***Step Type:*** | Flush |
| ***Step State:*** | Completed |
| ***State Change Cause:*** | EndProgram |
| ***Medication Reference ID:*** | 1984BYGO |
| ***Flush Order ID:*** | FLUSHIE2000 |
| ***Infusate Name:*** | Normal Saline |
| ***Drug Amt.:*** | ---- |
| ***Dil. Vol. (mL):*** | ---- |
| ***RATE (mL*/*h):*** | 1.3333 |
| ***VTBI (mL):*** | 0.5000 |
| ***Vol. Infused (mL):*** | 0.5000 |

12. Within the HIS, a clinician reviewing the I/O chart, such as via an eMAR, will see two entries: one entry for 2 mL of Clindamycin and one entry for 1 mL of the infusate named in the flush reports. The flush infusate will be positively and definitively associated with the primary administration of Clindamycin.

### Scenario 8 - Flush APR, First To Push Primed Volume

The syringe pump 30 may be configured to deliver a primary fluid delivery order and a flush delivery order using APRs. A dedicated medication line may be connected in standby to a 1 kg neonate. A time critical order of 0.2 mg of Indomethacin in 0.4 mL over 30 mins needs to be given ASAP. The tubing volume between pump and patient is 0.3 mL and the rate of the infusion is only 0.8 mL/h. The clinician loads the medication disposable and push 0.3 mL of Indomethacin to the bottom of the tubing in order to avoid any further delays in administration.

In this example, conditions may exist before and after executing the workflow.
- the pump module with dedicated medication line may be loaded with normal saline disposable but on standby for next medication order.
- For some pumps executing this workflow, once complete, the dedicated medication line may be left loaded with a normal saline disposable and returned to standby for next med order.

1. Unlike example Scenario 4, the clinician may cause an Indomethacin APR to be sent to the pump (or pump module). The APR may include a flush first step that specifies enough flush fluid volume so that the Indomethacin infusion will begin almost immediately. One example APR may be as follows:

| **APR Details** | **Flush Step** | **Order Step** |
|---|---|---|
| *Clinician ID:* | RatchedRN | |
| *Patient ID:* | W.Yueh | |
| *Order Type:* | PLUSH | Primary |
| *Order ID:* | FLUSH007PRN | MED42 |
| *Infusate Name:* | Normal Saline | Indomethacin |
| *Drug Amt:* | N/A | 0.2 mg |
| *Diluent Vol.:* | N/A | 0.4 |
| *Dose* | N/A | 0.2 mg/kg |
| *Rate:* | N/A | 0.8 |
| *VTBI:* | 0.3 | 0.4 |

The rate of the flush is shown in this example as N/A. However, in some implementations, the rate may be defined by one or more of: drug dose error reduction system (DERS); disposable limit; user or care area profile limit; specific limit in drug library (e.g., as flat value (rate/duration)); percent or other dynamic value based on the ordered medication rate (e.g., 10x ordered medication rate); or included in the flush step APR.
2. The pump or control unit associated with the pump module may prompt a clinician to load medication disposable
3. Once loaded, the pump or control unit or terminal may proceed to a Prime/Flush infusion step setup screen and display the following setup parameters:

| | |
|---|---|
| a. | Title Bar = Indomethacin - Prime/Flush |
| b. | Rate = 120 mL/h |
| c. | VTBI = 0.3 mL |
| d. | Duration = 15 seconds |
| e. | Avail. Vol. = 0.41 mL |

4. After or in conjunction with the Prime/Flush infusion step setup screen, the pump or control unit or terminal may present to a primary infusion step setup screen as follows:

| | |
|---|---|
| f. | Title Bar = Indomethacin |
| g. | Rate = 0.8 mL/h |
| h. | VTBI = 0.1 mL |
| i. | Duration = 30 minutes |
| j . | Avail. Vol. = 0.11 mL |

5. The VTBI may be dynamically generated value indicating the remaining dose volume after the prime/flush step. In this case, the value is Order VTBI (0.4) - Prime/Flush VTBI (0.1) to yield a value of 0.1mL.
6. In this example, the clinician reviews the parameters and selects START (e.g., activate a control element on a user interface, activate a softkey, activate an electromechanical button) to start the initial prime/flush.
7. The pump 30 starts priming at 120 mL/h and the pump or control unit or terminal associated with the pump, may display an incrementing prime volume, and report a prime start event to the HIS. The report may be one or more messages including information about the priming to the HIS. One example report may be as follows:

| ***Reporting Data*** | **Active Prime Step** |
|---|---|
| ***Infusion Event:*** | Start |
| ***Step Type:*** | PLUSH |
| ***Step State:*** | Infusing |
| ***State Change Cause:*** | InitStart |
| ***Medication Reference ID:*** | MED42 |
| ***Flush Order ID:*** | FLUSH007PRN |
| ***Infusate Name:*** | Normal Saline |
| ***Drug Amt.:*** | ---- |
| ***Dil. Vol. (mL):*** | ---- |
| ***RATE (mL*/*h):*** | 120.0000 |
| ***VTBI (mL):*** | 0.3000 |
| ***Vol. Primed (mL):*** | 0.0000 |

8.
A. When the volume infused counter reaches 0.3 mL, the pump 30 or control unit or terminal associated with the pump may present (e.g., on a display) a transition alert on the UI. One of these devices may also report a PLUSH transition event to the HIS. The report may be one or more messages including information about the infusion transmitted to a HIS. One example report may be as follows:

| ***Reporting Data*** | **Active Prime Step** |
|---|---|
| ***Infusion Event:*** | Stop |
| ***Step Type:*** | PLUSH |
| ***Step State:*** | Stop |
| ***State Change Cause:*** | SwitchSteps |
| ***Medication Reference ID:*** | MED42 |
| ***Flush Order ID:*** | FLUSH007PRN |
| ***Infusate Name:*** | Normal Saline |
| ***Drug Amt.:*** | ---- |
| ***Dil. Vol. (mL):*** | ---- |
| ***RATE (mL*/*h):*** | 120.0000 |
| ***VTBI (mL):*** | 0.3000 |
| ***Vol. Primed (mL):*** | 0.3000 |

The state change cause may be represented using a different state such as "switch containers". The selection of step state may be based on the original APR or subsequent parameters received at the pump or control unit or terminal.

B. The transition event report may be closely followed in time by transmission of a Start event indicating that the pump (or pump module) has started infusing the primary step, Indomethacin. The report may be one or more messages including information about the infusion to the HIS. One example report may be as follows:

| ***Reporting Data*** | **Active Primary Step** |
|---|---|
| ***Infusion Event:*** | Start |
| ***Step Type:*** | Intermittent |
| ***Step State:*** | Infusing |
| ***State Change Cause:*** | InitStart |
| ***Order ID:*** | MED42 |
| ***Infusate Name:*** | Indomethacin |
| ***Drug Amt.:*** | 0.2000 mg |
| ***Dil. Vol. (mL):*** | 0.4000 |
| ***RATE (mL*/*h):*** | 0.8000 |
| ***VTBI (mL):*** | 0.1000 |
| ***Vol. Infused (mL):*** | 0.0000 |

9. Once the volume of 0.1 mL is infused, the pump (or pump module) may stop and report (directly or via the control unit or terminal) an infusion complete event. The report may be one or more messages including information about the infusion transmitted to a HIS. One example report may be as follows:

| ***Reporting Data*** | **Active Primary Step** |
|---|---|
| ***Infusion Event:*** | Complete |
| ***Step Type:*** | Intermittent |
| ***Step State:*** | Completed |
| ***State Change Cause:*** | EndProgram |
| ***Order ID:*** | MED42 |
| ***Infusate Name:*** | Indomethacin |
| ***Drug Amt.:*** | 0.2000 mg |
| ***Dil. Vol. (mL):*** | 0.4000 |
| ***RATE (mL*/*h):*** | 0.8000 |
| ***VTBI (mL):*** | 0.1000 |
| ***Vol. Infused (mL):*** | 0.1000 |

10. Clinician may load another flush syringe to complete the dose to the patient and follows another workflow, such as discussed in example Scenario 1 to complete dose and return the medication line back to a standby state.
11. Within the HIS, a clinician may see two entries one corresponding to the 0.4 mL volume infused of Indomethacin in eMAR and one corresponding to the 0.3 mL as generic flush. The clinician may be prompted to document 0.3 mL of Normal Saline for the Indomethacin prime volume.
12. Using the HIS, the clinician may associate the 0.3 mL of volume to PRN flush order in the eMAR.

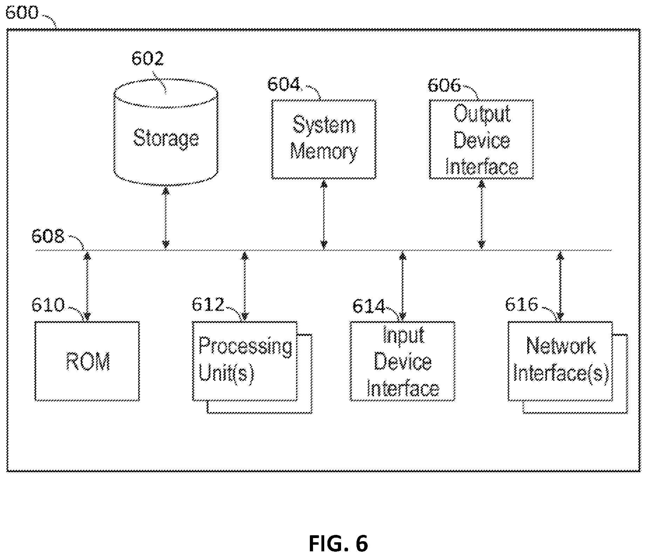

FIG. 6 is a conceptual diagram illustrating an example electronic system 600 for accurate delivery of a flush infusion, according to aspects of the subj ect technology. Electronic system 600 may be a computing device for execution of software associated with one or more portions or steps of method 600, or components and methods provided by FIGS. 1-5, including but not limited to computing hardware within medical device 122, or syringe pump 30, control unit 14, and/or any computing devices or associated terminals disclosed herein. In this regard, electronic system 600 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 600 may include various types of computer readable media and interfaces for various other types of computer readable media. In the depicted example, electronic system 600 includes a bus 608, processing unit(s) 612, a system memory 604, a read-only memory (ROM) 610, a permanent storage device 602, an input device interface 614, an output device interface 606, and one or more network interfaces 616. In some implementations, electronic system 600 may include or be integrated with other computing devices or circuitry for operation of the various components and methods previously described.

Bus 608 collectively represents all system, peripheral, and chipset buses that communicatively connect the numerous internal devices of electronic system 600. For instance, bus 408 communicatively connects processing unit(s) 612 with ROM 610, system memory 604, and permanent storage device 602.

From these various memory units, processing unit(s) 612 retrieves instructions to execute and data to process in order to execute the processes of the subject disclosure. The processing unit(s) can be a single processor or a multi-core processor in different implementations.

ROM 610 stores static data and instructions that are needed by processing unit(s) 612 and other modules of the electronic system. Permanent storage device 602, on the other hand, is a read-and-write memory device. This device is a non-volatile memory unit that stores instructions and data even when electronic system 600 is off. Some implementations of the subject disclosure use a mass-storage device (such as a magnetic or optical disk and its corresponding disk drive) as permanent storage device 602.

Other implementations use a removable storage device (such as a floppy disk, flash drive, and its corresponding disk drive) as permanent storage device 602. Like permanent storage device 602, system memory 604 is a read-and-write memory device. However, unlike storage device 602, system memory 604 is a volatile read-and-write memory, such a random access memory. System memory 604 stores some of the instructions and data that the processor needs at runtime. In some implementations, the processes of the subject disclosure are stored in system memory 604, permanent storage device 602, and/or ROM 610. From these various memory units, processing unit(s) 612 retrieves instructions to execute and data to process in order to execute the processes of some implementations.

Bus 608 also connects to input and output device interfaces 614 and 606. Input device interface 614 enables the user to communicate information and select commands to the electronic system. Input devices used with input device interface 614 include, e.g., alphanumeric keyboards and pointing devices (also called "cursor control devices"). Output device interfaces 606 enables, e.g., the display of images generated by the electronic system 600. Output devices used with output device interface 606 include, e.g., printers and display devices, such as cathode ray tubes (CRT) or liquid crystal displays (LCD). Some implementations include devices such as a touchscreen that functions as both input and output devices.

Also, as shown in FIG. 6, bus 608 also couples electronic system 600 to a network (not shown) through network interfaces 616. Network interfaces 616 may include, e.g., a wireless access point (e.g., Bluetooth or WiFi) or radio circuitry for connecting to a wireless access point. Network interfaces 616 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 600 can be used in conjunction with the subject disclosure.

These functions described above can be implemented in computer software, firmware or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some embodiments, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

### Further Consideration:

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The predicate words "configured to", "operable to", and "programmed to" do not imply any particular tangible or intangible modification of a subject, but, rather, are intended to be used interchangeably. For example, a processor configured to monitor and control an operation or a component may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such as an "embodiment" may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some embodiments, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine readable aggregation of information such as an XML document, fixed field message, comma separated message, JSON, a custom protocol, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In any embodiment, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. A system, comprising:
a syringe pump comprising a receptacle for receiving a syringe;
one or more sensors configured to generate sensor data pertaining to the syringe within the receptacle of the syringe pump; and
one or more processors configured to execute instructions and perform operations, comprising:
receiving the sensor data indicating a new syringe being loaded into the syringe pump;
determining, after receiving the sensor data and based on electronically stored information associated with the syringe pump, that the syringe pump is currently associated with a first fluid delivery order for a predetermined amount of a first fluid, and that at least a first portion of the first fluid was previously delivered by the syringe pump according to the first fluid delivery order before the new syringe was loaded into the syringe pump;
determining a second portion of the first fluid remaining to be delivered to complete the predetermined amount of the first fluid for the first fluid delivery order, the second portion of the first fluid being in a downstream fluid administration line;
initiating delivery of a second fluid from the new syringe by the syringe pump according to a second fluid delivery order, wherein the syringe pump is caused to deliver the second fluid by driving a motor of the syringe pump responsive to initiating the delivery of the second fluid from the new syringe, and continues the delivery of the second fluid from the new syringe by driving the motor of the syringe pump; and
when an amount of the second fluid delivered from the new syringe satisfies the second portion of the first fluid remaining to be delivered, automatically, without user intervention and without stopping the motor:
electronically indicating the first fluid delivery order as being complete,
de-associating the first fluid delivery order from the syringe pump,
associating the syringe pump with the second fluid delivery order,
generating a start condition for the second delivery order, wherein the start condition for the second fluid delivery order is not generated until only after the first fluid delivery order is determined to be complete by way of the second remaining portion having been purged from the downstream fluid administration line, and
continuing delivery of the second fluid from the new syringe.

2. The system of Claim 1, the operations further comprising:
receiving, before the new syringe is loaded into the syringe pump, an indication of a first syringe loaded in the syringe pump being emptied;
generate, based on receiving the indication of the first syringe being emptied, an empty condition for the first fluid delivery order;
generating a restart condition for the first fluid delivery order contemporaneously with initiating the delivery of the second fluid according to the second fluid delivery order; and
generating a start condition for the second fluid delivery order contemporaneously with associating the syringe pump with the second fluid delivery order to continue delivery of the second fluid from the new syringe.

3. The system of Claim 2, wherein delivery of the first fluid and the second fluid is measured in volumetric units, the operations further comprising:
continuously measuring, for the first fluid delivery order, a total volume of the first fluid over a delivery of a portion of the first fluid from the first syringe before the empty condition is generated and through the delivery of the second fluid from the new syringe until the amount of the second fluid delivered from the new syringe satisfies the second portion of the first fluid remaining to be delivered;
confirming that the measured total volume of the first fluid was delivered according to the first fluid delivery order; and
providing the total volume for display on a display device.

4. The system of Claim 2 or Claim 3, wherein the first fluid is a medication and the second fluid is a non-medicinal flush infusate, the operations further comprising:
causing the syringe pump to deliver a portion of the first fluid from the first syringe until the indication of the first syringe being emptied is received;
pausing the first fluid delivery order before the delivery of the second fluid is initiated, wherein, when the first syringe is emptied and the delivery of the second fluid is initiated, the second portion of the first fluid remaining to be delivered is residing in an infusion line connected to the syringe pump but not delivered to a patient; and
causing, responsive to initiating delivery of the second fluid, the syringe pump to deliver the portion of first fluid from the infusion line by way of pushing the portion of the first fluid through the infusion line with the second fluid.

5. The system of Claim 4, the operations further comprising:
receiving, from a remote computing system, an automated programming command to configure the syringe pump to deliver the non-medicinal flush infusate according to the
second fluid delivery order; and
responsive to receiving the automated programming command, without user intervention:
disabling a syringe empty alarm currently set to be provided when the first syringe loaded in the syringe pump is emptied; and
automatically programming the syringe pump to initiate the delivery of the second fluid from the new syringe by the syringe pump according to the second fluid delivery order.

6. The system of Claim 5, wherein the automated programming command causes the
syringe pump to:
automatically programming the syringe pump to initiate the delivery of the first fluid from the first syringe by the syringe pump according to the first fluid delivery order;
prompt, when the first syringe is emptied, a user to unload the first syringe and to load the new syringe; and
automatically stop delivery of the second fluid when the amount of the second fluid delivered from the new syringe satisfies the second portion of the first fluid remaining to be delivered.

7. The system of any one of Claims 2 through 6, wherein indicating the first fluid delivery order as being complete comprises:
associating a type of the second fluid and a flush complete event with the first fluid delivery order.

8. The system of any one of Claims 2 through 6, the operations further comprising:
receiving, based on the generated sensor data, an indication of the new syringe being emptied; and
providing an indication that the second fluid delivery order is complete when the indication of the new syringe being emptied is received.

9. The system of any one of Claims 1 through 8, the operations further comprising, when the amount of the second fluid delivered from the new syringe satisfies the second portion of the first fluid remaining to be delivered, automatically, without user intervention:
prompting a user for a confirmation to continue delivery of the second fluid from the new syringe; and
continuing delivery of the second fluid from the new syringe responsive to receiving the confirmation.

10. A non-transitory machine-readable storage medium embodying instructions that, when executed by a machine, cause the machine to perform a method comprising:
receiving, from one or more sensors associated with a syringe pump, an indication of a new syringe being loaded into the syringe pump;
determining, after receiving the indication based on electronically stored information associated with the syringe pump, that the syringe pump is currently associated with a first fluid delivery order for a predetermined amount of a first fluid, and that at least a first portion of the first fluid was previously delivered by the syringe pump according to the first fluid delivery order before the new syringe was loaded into the syringe pump;
determining a second portion of the first fluid remaining to be delivered to complete the predetermined amount of the first fluid for the first fluid delivery order, the second portion of the first fluid being in a downstream fluid administration line;
initiating delivery of a second fluid from the new syringe by the syringe pump according to a second fluid delivery order, wherein the syringe pump is caused to deliver the second fluid by driving a motor of the syringe pump responsive to initiating the delivery of the second fluid from the new syringe, and continues the delivery of the second fluid from the new syringe by driving the motor of the syringe pump; and
when an amount of the second fluid delivered from the new syringe satisfies the second portion of the first fluid remaining to be delivered, automatically, without user intervention and without stopping the motor:
electronically indicating the first fluid delivery order as being complete,
de-associating the first fluid delivery order from the syringe pump,
associating the syringe pump with the second fluid delivery order,
generating a start condition for the second delivery order, wherein the start condition for the second fluid delivery order is not generated until only after the first fluid delivery order is determined to be complete by way of the second remaining portion having been purged from the downstream fluid administration line, and
continuing delivery of the second fluid from the new syringe.

11. The non-transitory machine-readable medium of Claim 10, the method further comprising:
receiving, before the new syringe is loaded into the syringe pump, an indication of a first syringe loaded in the syringe pump being emptied;
generate, based on receiving the indication of the first syringe being emptied, an empty condition for the first fluid delivery order;
generating a restart condition for the first fluid delivery order contemporaneously with initiating the delivery of the second fluid according to the second fluid delivery order; and generating a start condition for the second fluid delivery order contemporaneously with associating the syringe pump with the second fluid delivery order to continue delivery of the second fluid from the new syringe.

12. The non-transitory machine-readable medium of Claim 11, wherein delivery of the first fluid and the second fluid is measured in volumetric units, the method further comprising:
continuously measuring, for the first fluid delivery order, a total volume of the first fluid over a delivery of the first fluid from the first syringe before the empty condition is generated and through the delivery of the second fluid from the new syringe until the amount of the second fluid delivered from the new syringe satisfies the second portion of the first fluid remaining to be delivered;
confirming that the measured total volume of the first fluid was delivered according to the first fluid delivery order; and
providing the total volume for display on a display device.

13. The non-transitory machine-readable medium of Claim 11 or Claim 12, wherein the first fluid is a medication and the second fluid is a non-medicinal flush infusate, the method further comprising:
causing the syringe pump to deliver a portion of the first fluid from the first syringe until the indication of the first syringe being emptied is received;
pausing the first fluid delivery order before the delivery of the second fluid is initiated, wherein, when the first syringe is emptied and the delivery of the second fluid is initiated, the second portion of the first fluid remaining to be delivered is residing in an infusion line connected to the syringe pump but not delivered to a patient; and
causing, responsive to initiating delivery of the second fluid, the syringe pump to deliver the portion of first fluid from the infusion line by way of pushing the portion of the
first fluid through the infusion line with the second fluid.

14. The non-transitory machine-readable medium of Claim 13, the method further comprising:
receiving, from a remote computing system, an automated programming command to configure the syringe pump to deliver the non-medicinal flush infusate according to the second fluid delivery order; and
responsive to receiving the automated programming command, without user intervention:
disabling a syringe empty alarm currently set to be provided when the first syringe loaded in the syringe pump is emptied; and
automatically programming the syringe pump to initiate the delivery of the second fluid from the new syringe by the syringe pump according to the second fluid delivery order.

15. The non-transitory machine-readable medium of Claim 14, wherein the automated programming command causes the syringe pump to:
automatically program the syringe pump to initiate the delivery of the first fluid from the first syringe by the syringe pump according to the first fluid delivery order;
prompt, when the first syringe is emptied, a user to unload the first syringe and to load the new syringe; and
automatically stop delivery of the second fluid when the amount of the second fluid delivered from the new syringe satisfies the second portion of the first fluid remaining to be delivered.

16. The non-transitory machine-readable medium of any one of Claims 10 through 15, wherein indicating the first fluid delivery order as being complete comprises:
associating a type of the second fluid and a flush complete event with the first fluid delivery order.

17. The non-transitory machine-readable medium of any one of Claims 10 through 15, the method further comprising:
receiving an indication of the new syringe being emptied; and
providing an indication that the second fluid delivery order is complete when the indication of the new syringe being emptied is received.

18. The non-transitory machine-readable medium of any one of Claims 10 through 17, the method further comprising, when the amount of the second fluid delivered from the new syringe satisfies the second portion of the first fluid remaining to be delivered, automatically, without user intervention:
prompting a user for a confirmation to continue delivery of the second fluid from the new syringe; and
continuing delivery of the second fluid from the new syringe responsive to receiving the confirmation.

## Patentansprüche

1. System, aufweisend:
eine Spritzenpumpe mit einer Aufnahme zum Aufnehmen einer Spritze;
einen oder mehrere Sensoren, die dazu konfiguriert sind, Sensordaten in Bezug auf die Spritze innerhalb der Aufnahme der Spritzenpumpe zu erzeugen; und
einen oder mehrere Prozessoren, die dazu konfiguriert sind, Anweisungen auszuführen und Vorgänge durchzuführen, umfassend:
Empfangen der Sensordaten, die anzeigen, dass eine neue Spritze in die Spritzenpumpe geladen wird;
Bestimmen, nach dem Empfangen der Sensordaten und auf der Grundlage elektronisch gespeicherter Informationen, die der Spritzenpumpe zugeordnet sind, dass die Spritzenpumpe derzeit einem ersten Fluidabgabeauftrag für eine vorbestimmte Menge eines ersten Fluids zugeordnet ist und dass zumindest ein erster Teil des ersten Fluids zuvor von der Spritzenpumpe gemäß dem ersten Fluidabgabeauftrag abgegeben wurde, bevor die neue Spritze in die Spritzenpumpe geladen wurde;
Bestimmen eines zweiten Teils des ersten Fluids, der noch abzugeben ist, um die vorbestimmte Menge des ersten Fluids für den ersten Fluidabgabeauftrag zu vervollständigen, wobei sich der zweite Teil des ersten Fluids in einer stromabwärts gelegenen Fluidverabreichungsleitung befindet;
Initiieren der Abgabe eines zweiten Fluids aus der neuen Spritze durch die Spritzenpumpe gemäß einem zweiten Fluidabgabeauftrag, wobei die Spritzenpumpe dazu veranlasst wird, das zweite Fluid durch Antreiben eines Motors der Spritzenpumpe als Reaktion auf das Initiieren der Abgabe des zweiten Fluids aus der neuen Spritze abzugeben, und die Abgabe des zweiten Fluids aus der neuen Spritze durch Antreiben des Motors der Spritzenpumpe fortsetzt; und
wenn eine Menge des aus der neuen Spritze abgegebenen zweiten Fluids den zweiten Teil des noch abzugebenden ersten Fluids erfüllt, automatisch, ohne Benutzereingriff und ohne Anhalten des Motors:
elektronisches Anzeigen, dass der erste Fluidabgabeauftrag abgeschlossen ist,
Aufheben der Zuordnung des ersten Fluidabgabeauftrags zu der Spritzenpumpe,
Zuordnen der Spritzenpumpe zu dem zweiten Fluidabgabeauftrag,
Erzeugen einer Startbedingung für den zweiten Abgabeauftrag, wobei die Startbedingung für den zweiten Fluidabgabeauftrag erst dann erzeugt wird, nachdem der erste Fluidabgabeauftrag dadurch als abgeschlossen bestimmt wurde, dass der zweite verbleibende Teil aus der stromabwärts gelegenen Fluidverabreichungsleitung ausgespült wurde, und
Fortsetzen der Abgabe des zweiten Fluids aus der neuen Spritze.

2. System nach Anspruch 1, wobei die Vorgänge ferner umfassen: Empfangen, bevor die neue Spritze in die Spritzenpumpe geladen wird, einer Anzeige, dass eine erste in der Spritzenpumpe geladene Spritze entleert ist; Erzeugen, basierend auf dem Empfangen der Anzeige des Entleerens der ersten Spritze, einer Leerbedingung für den ersten Fluidabgabeauftrag; Erzeugen einer Neustartbedingung für den ersten Fluidabgabeauftrag zeitgleich mit dem Initiieren der Abgabe des zweiten Fluids gemäß dem zweiten Fluidabgabeauftrag; und Erzeugen einer Startbedingung für den zweiten Fluidabgabeauftrag zeitgleich mit dem Zuordnen der Spritzenpumpe zu dem zweiten Fluidabgabeauftrag, um die Abgabe des zweiten Fluids aus der neuen Spritze fortzusetzen.

3. System nach Anspruch 2, wobei die Abgabe des ersten Fluids und des zweiten Fluids in volumetrischen Einheiten gemessen wird, wobei die Vorgänge ferner umfassen:
kontinuierliches Messen, für den ersten Fluidabgabeauftrag, eines Gesamtvolumens des ersten Fluids über eine Abgabe eines Teils des ersten Fluids aus der ersten Spritze vor dem Erzeugen der Leerbedingung und durch die Abgabe des zweiten Fluids aus der neuen Spritze hindurch, bis die Menge des aus der neuen Spritze abgegebenen zweiten Fluids den zweiten Teil des noch abzugebenden ersten Fluids erfüllt;
Bestätigen, dass das gemessene Gesamtvolumen des ersten Fluids gemäß dem ersten Fluidabgabeauftrag abgegeben wurde; und
Bereitstellen des Gesamtvolumens zur Anzeige auf einer Anzeigeeinrichtung.

4. System nach Anspruch 2 oder 3, wobei das erste Fluid ein Medikament und das zweite Fluid eine nicht-medikamentöse Spüllösung ist, wobei die Vorgänge ferner umfassen:
Veranlassen der Spritzenpumpe, einen Teil des ersten Fluids aus der ersten Spritze abzugeben, bis die Anzeige des Entleerens der ersten Spritze empfangen wird;
Pausieren des ersten Fluidabgabeauftrags vor dem Initiieren der Abgabe des zweiten Fluids, wobei, wenn die erste Spritze entleert ist und die Abgabe des zweiten Fluids initiiert wird, der zweite Teil des noch abzugebenden ersten Fluids in einer mit der Spritzenpumpe verbundenen Infusionsleitung verbleibt, jedoch nicht an einen Patienten verabreicht wird; und
Veranlassen der Spritzenpumpe, als Reaktion auf das Initiieren der Abgabe des zweiten Fluids den Teil des ersten Fluids aus der Infusionsleitung abzugeben, indem der Teil des ersten Fluids mit dem zweiten Fluid durch die Infusionsleitung gedrückt wird.

5. System nach Anspruch 4, wobei die Vorgänge ferner umfassen:
Empfangen, von einem entfernten Computersystem, eines automatisierten Programmierbefehls zur Konfiguration der Spritzenpumpe zur Abgabe der nicht-medikamentösen Spüllösung gemäß dem zweiten Fluidabgabeauftrag; und
als Reaktion auf das Empfangen des automatisierten Programmierbefehls, ohne Benutzereingriff:
Deaktivieren eines Spritzenleeralarms, der derzeit so eingestellt ist, dass er beim Entleeren der ersten in der Spritzenpumpe geladenen Spritze ausgegeben wird; und
automatisches Programmieren der Spritzenpumpe, um die Abgabe des zweiten Fluids aus der neuen Spritze durch die Spritzenpumpe gemäß dem zweiten Fluidabgabeauftrag zu initiieren.

6. System nach Anspruch 5, wobei der automatisierte Programmierbefehl bewirkt, dass die Spritzenpumpe:
automatisch so programmiert wird, dass sie die Abgabe des ersten Fluids aus der ersten Spritze gemäß dem ersten Fluidabgabeauftrag initiiert;
einen Benutzer auffordert, wenn die erste Spritze entleert ist, die erste Spritze zu entladen und die neue Spritze zu laden; und
die Abgabe des zweiten Fluids automatisch stoppt, wenn die Menge des aus der neuen Spritze abgegebenen zweiten Fluids den zweiten Teil des noch abzugebenden ersten Fluids erfüllt.

7. System nach einem der Ansprüche 2 bis 6, wobei das Anzeigen, dass der erste Fluidabgabeauftrag abgeschlossen ist, umfasst:
Zuordnen eines Typs des zweiten Fluids und eines Spülabschlussereignisses zu dem ersten Fluidabgabeauftrag.

8. System nach einem der Ansprüche 2 bis 6, wobei die Vorgänge ferner umfassen: Empfangen, basierend auf den erzeugten Sensordaten, einer Anzeige, dass die neue Spritze entleert ist; und Bereitstellen einer Anzeige, dass der zweite Fluidabgabeauftrag abgeschlossen ist, wenn die Anzeige des Entleerens der neuen Spritze empfangen wird.

9. System nach einem der Ansprüche 1 bis 8, wobei die Vorgänge ferner umfassen, wenn die Menge des aus der neuen Spritze abgegebenen zweiten Fluids den zweiten Teil des noch abzugebenden ersten Fluids erfüllt, automatisch, ohne Benutzereingriff:
Auffordern eines Benutzers zur Bestätigung, die Abgabe des zweiten Fluids aus der neuen Spritze fortzusetzen; und
Fortsetzen der Abgabe des zweiten Fluids aus der neuen Spritze als Reaktion auf den Empfang der Bestätigung.

10. Nicht-flüchtiges maschinenlesbares Speichermedium, das Anweisungen verkörpert, die, wenn sie von einer Maschine ausgeführt werden, die Maschine veranlassen, ein Verfahren durchzuführen, umfassend:
Empfangen, von einem oder mehreren Sensoren, die einer Spritzenpumpe zugeordnet sind, einer Anzeige, dass eine neue Spritze in die Spritzenpumpe geladen wird;
Bestimmen, nach dem Empfangen der Anzeige auf der Grundlage elektronisch gespeicherter Informationen, die der Spritzenpumpe zugeordnet sind, dass die Spritzenpumpe derzeit einem ersten Fluidabgabeauftrag für eine vorbestimmte Menge eines ersten Fluids zugeordnet ist und dass zumindest ein erster Teil des ersten Fluids zuvor von der Spritzenpumpe gemäß dem ersten Fluidabgabeauftrag abgegeben wurde, bevor die neue Spritze in die Spritzenpumpe geladen wurde;
Bestimmen eines zweiten Teils des ersten Fluids, der noch abzugeben ist, um die vorbestimmte Menge des ersten Fluids für den ersten Fluidabgabeauftrag zu vervollständigen, wobei sich der zweite Teil des ersten Fluids in einer stromabwärts gelegenen Fluidverabreichungsleitung befindet;
Initiieren der Abgabe eines zweiten Fluids aus der neuen Spritze durch die Spritzenpumpe gemäß einem zweiten Fluidabgabeauftrag, wobei die Spritzenpumpe dazu veranlasst wird, das zweite Fluid durch Antreiben eines Motors der Spritzenpumpe als Reaktion auf das Initiieren der Abgabe des zweiten Fluids aus der neuen Spritze abzugeben, und die Abgabe des zweiten Fluids aus der neuen Spritze durch Antreiben des Motors der Spritzenpumpe fortsetzt; und
wenn eine Menge des aus der neuen Spritze abgegebenen zweiten Fluids den zweiten Teil des noch abzugebenden ersten Fluids erfüllt, automatisch, ohne Benutzereingriff und ohne Anhalten des Motors:
elektronisches Anzeigen, dass der erste Fluidabgabeauftrag abgeschlossen ist,
Aufheben der Zuordnung des ersten Fluidabgabeauftrags zu der Spritzenpumpe,
Zuordnen der Spritzenpumpe zu dem zweiten Fluidabgabeauftrag,
Erzeugen einer Startbedingung für den zweiten Abgabeauftrag, wobei die Startbedingung für den zweiten Fluidabgabeauftrag erst dann erzeugt wird, nachdem der erste Fluidabgabeauftrag dadurch als abgeschlossen bestimmt wurde, dass der zweite verbleibende Teil aus der stromabwärts gelegenen Fluidverabreichungsleitung ausgespült wurde, und
Fortsetzen der Abgabe des zweiten Fluids aus der neuen Spritze.

11. Nicht-flüchtiges maschinenlesbares Medium nach Anspruch 10, wobei das Verfahren ferner umfasst: Empfangen, bevor die neue Spritze in die Spritzenpumpe geladen wird, einer Anzeige, dass eine erste in der Spritzenpumpe geladene Spritze entleert ist; Erzeugen, basierend auf dem Empfangen der Anzeige des Entleerens der ersten Spritze, einer Leerbedingung für den ersten Fluidabgabeauftrag; Erzeugen einer Neustartbedingung für den ersten Fluidabgabeauftrag zeitgleich mit dem Initiieren der Abgabe des zweiten Fluids gemäß dem zweiten Fluidabgabeauftrag; und Erzeugen einer Startbedingung für den zweiten Fluidabgabeauftrag zeitgleich mit dem Zuordnen der Spritzenpumpe zu dem zweiten Fluidabgabeauftrag, um die Abgabe des zweiten Fluids aus der neuen Spritze fortzusetzen.

12. Nicht-flüchtiges maschinenlesbares Medium nach Anspruch 11, wobei die Abgabe des ersten Fluids und des zweiten Fluids in volumetrischen Einheiten gemessen wird, wobei das Verfahren ferner umfasst:
kontinuierliches Messen, für den ersten Fluidabgabeauftrag, eines Gesamtvolumens des ersten Fluids über die Abgabe des ersten Fluids aus der ersten Spritze vor dem Erzeugen der Leerbedingung und durch die Abgabe des zweiten Fluids aus der neuen Spritze hindurch, bis die Menge des aus der neuen Spritze abgegebenen zweiten Fluids den zweiten Teil des noch abzugebenden ersten Fluids erfüllt;
Bestätigen, dass das gemessene Gesamtvolumen des ersten Fluids gemäß dem ersten Fluidabgabeauftrag abgegeben wurde; und
Bereitstellen des Gesamtvolumens zur Anzeige auf einer Anzeigeeinrichtung.

13. Nicht-flüchtiges maschinenlesbares Medium nach Anspruch 11 oder 12, wobei das erste Fluid ein Medikament und das zweite Fluid eine nicht-medikamentöse Spüllösung ist, wobei das Verfahren ferner umfasst:
Veranlassen der Spritzenpumpe, einen Teil des ersten Fluids aus der ersten Spritze abzugeben, bis die Anzeige des Entleerens der ersten Spritze empfangen wird;
Pausieren des ersten Fluidabgabeauftrags vor dem Initiieren der Abgabe des zweiten Fluids, wobei, wenn die erste Spritze entleert ist und die Abgabe des zweiten Fluids initiiert wird, der zweite Teil des noch abzugebenden ersten Fluids in einer mit der Spritzenpumpe verbundenen Infusionsleitung verbleibt, jedoch nicht an einen Patienten verabreicht wird; und
Veranlassen der Spritzenpumpe, als Reaktion auf das Initiieren der Abgabe des zweiten Fluids den Teil des ersten Fluids aus der Infusionsleitung abzugeben, indem der Teil des ersten Fluids mit dem zweiten Fluid durch die Infusionsleitung gedrückt wird.

14. Nicht-flüchtiges maschinenlesbares Medium nach Anspruch 13, wobei das Verfahren ferner umfasst: Empfangen, von einem entfernten Computersystem, eines automatisierten Programmierbefehls zur Konfiguration der Spritzenpumpe zur Abgabe der nicht-medikamentösen Spüllösung gemäß dem zweiten Fluidabgabeauftrag; und
als Reaktion auf das Empfangen des automatisierten Programmierbefehls, ohne Benutzereingriff:
Deaktivieren eines Spritzenleeralarms, der derzeit so eingestellt ist, dass er beim Entleeren der ersten in der Spritzenpumpe geladenen Spritze ausgegeben wird; und
automatisches Programmieren der Spritzenpumpe, um die Abgabe des zweiten Fluids aus der neuen Spritze durch die Spritzenpumpe gemäß dem zweiten Fluidabgabeauftrag zu initiieren.

15. Nicht-flüchtiges maschinenlesbares Medium nach Anspruch 14, wobei der automatisierte Programmierbefehl bewirkt, dass die Spritzenpumpe:
automatisch so programmiert wird, dass sie die Abgabe des ersten Fluids aus der ersten Spritze gemäß dem ersten Fluidabgabeauftrag initiiert;
einen Benutzer auffordert, wenn die erste Spritze entleert ist, die erste Spritze zu entladen und die neue Spritze zu laden; und
die Abgabe des zweiten Fluids automatisch stoppt, wenn die Menge des aus der neuen Spritze abgegebenen zweiten Fluids den zweiten Teil des noch abzugebenden ersten Fluids erfüllt.

16. Nicht-flüchtiges maschinenlesbares Medium nach einem der Ansprüche 10 bis 15, wobei das Anzeigen, dass der erste Fluidabgabeauftrag abgeschlossen ist, umfasst:
Zuordnen eines Typs des zweiten Fluids und eines Spülabschlussereignisses zu dem ersten Fluidabgabeauftrag.

17. Nicht-flüchtiges maschinenlesbares Medium nach einem der Ansprüche 10 bis 15, wobei das Verfahren ferner umfasst: Empfangen einer Anzeige, dass die neue Spritze entleert ist; und Bereitstellen einer Anzeige, dass der zweite Fluidabgabeauftrag abgeschlossen ist, wenn die Anzeige des Entleerens der neuen Spritze empfangen wird.

18. Nicht-flüchtiges maschinenlesbares Medium nach einem der Ansprüche 10 bis 17, wobei das Verfahren ferner umfasst, wenn die Menge des aus der neuen Spritze abgegebenen zweiten Fluids den zweiten Teil des noch abzugebenden ersten Fluids erfüllt, automatisch, ohne Benutzereingriff:
Auffordern eines Benutzers zur Bestätigung, die Abgabe des zweiten Fluids aus der neuen Spritze fortzusetzen; und
Fortsetzen der Abgabe des zweiten Fluids aus der neuen Spritze als Reaktion auf den Empfang der Bestätigung.

## Revendications

1. Système comprenant :
une pompe à seringue comprenant un réceptacle destiné à recevoir une seringue ;
un ou plusieurs capteurs configurés pour générer des données de capteur relatives à la seringue dans le réceptacle de la pompe à seringue ; et
un ou plusieurs processeurs configurés pour exécuter des instructions et effectuer des opérations, comprenant :
la réception des données de capteur indiquant qu'une nouvelle seringue est chargée dans la pompe à seringue ;
la détermination, après réception des données de capteur et sur la base d'informations stockées électroniquement associées à la pompe à seringue, que la pompe à seringue est actuellement associée à un premier ordre d'administration de fluide pour une quantité prédéterminée d'un premier fluide, et qu'au moins une première portion du premier fluide a été précédemment administrée par la pompe à seringue conformément au premier ordre d'administration de fluide avant que la nouvelle seringue ne soit chargée dans la pompe à seringue ;
la détermination d'une seconde portion du premier fluide restant à administrer pour compléter la quantité prédéterminée du premier fluide pour le premier ordre d'administration de fluide, la seconde portion du premier fluide se trouvant dans une ligne d'administration de fluide en aval ;
le lancement de l'administration d'un second fluide à partir de la nouvelle seringue par la pompe à seringue conformément à un second ordre d'administration de fluide, la pompe à seringue étant amenée à administrer le second fluide par l'entraînement d'un moteur de la pompe à seringue en réponse au lancement de l'administration du second fluide à partir de la nouvelle seringue, et poursuivant l'administration du second fluide à partir de la nouvelle seringue par l'entraînement du moteur de la pompe à seringue; et
lorsqu'une quantité du second fluide administrée à partir de la nouvelle seringue satisfait la seconde portion du premier fluide restant à administrer, automatiquement, sans intervention de l'utilisateur et sans arrêter le moteur :
l'indication électronique que le premier ordre d'administration de fluide est terminé,
la dissociation du premier ordre d'administration de fluide de la pompe à seringue,
l'association de la pompe à seringue au second ordre d'administration de fluide,
la génération d'une condition de démarrage pour le second ordre d'administration, la condition de démarrage pour le second ordre d'administration de fluide n'étant générée qu'après que le premier ordre d'administration de fluide a été déterminé comme terminé du fait que la seconde portion restante a été purgée de la ligne d'administration de fluide en aval, et
la poursuite de l'administration du second fluide à partir de la nouvelle seringue.

2. Système selon la revendication 1, les opérations comprenant en outre : la réception, avant que la nouvelle seringue ne soit chargée dans la pompe à seringue, d'une indication qu'une première seringue chargée dans la pompe à seringue est vidée ; la génération, sur la base de la réception de l'indication de vidange de la première seringue, d'une condition de vide pour le premier ordre d'administration de fluide ; la génération d'une condition de redémarrage pour le premier ordre d'administration de fluide simultanément au lancement de l'administration du second fluide conformément au second ordre d'administration de fluide ; et la génération d'une condition de démarrage pour le second ordre d'administration de fluide simultanément à l'association de la pompe à seringue au second ordre d'administration de fluide afin de poursuivre l'administration du second fluide à partir de la nouvelle seringue.

3. Système selon la revendication 2, dans lequel l'administration du premier fluide et du second fluide est mesurée en unités volumétriques, les opérations comprenant en outre :
la mesure continue, pour le premier ordre d'administration de fluide, d'un volume total du premier fluide sur l'administration d'une portion du premier fluide à partir de la première seringue avant la génération de la condition de vide et à travers l'administration du second fluide à partir de la nouvelle seringue jusqu'à ce que la quantité du second fluide administrée à partir de la nouvelle seringue satisfasse la seconde portion du premier fluide restant à administrer ;
la confirmation que le volume total mesuré du premier fluide a été administré conformément au premier ordre d'administration de fluide ; et
la fourniture du volume total pour affichage sur un dispositif d'affichage.

4. Système selon la revendication 2 ou 3, dans lequel le premier fluide est un médicament et le second fluide est un liquide de rinçage non médicamenteux, les opérations comprenant en outre :
le fait de faire administrer par la pompe à seringue une portion du premier fluide à partir de la première seringue jusqu'à la réception de l'indication de vidange de la première seringue ;
la mise en pause du premier ordre d'administration de fluide avant le lancement de l'administration du second fluide, dans lequel, lorsque la première seringue est vidée et que l'administration du second fluide est lancée, la seconde portion du premier fluide restant à administrer réside dans une ligne de perfusion connectée à la pompe à seringue mais n'est pas administrée à un patient ; et
le fait de faire administrer par la pompe à seringue, en réponse au lancement de l'administration du second fluide, la portion du premier fluide depuis la ligne de perfusion en poussant la portion du premier fluide à travers la ligne de perfusion avec le second fluide.

5. Système selon la revendication 4, les opérations comprenant en outre :
la réception, depuis un système informatique distant, d'une commande de programmation automatisée pour configurer la pompe à seringue afin d'administrer le liquide de rinçage non médicamenteux conformément au second ordre d'administration de fluide ; et
en réponse à la réception de la commande de programmation automatisée, sans intervention de l'utilisateur :
la désactivation d'une alarme de seringue vide actuellement configurée pour être fournie lorsque la première seringue chargée dans la pompe à seringue est vidée ; et
la programmation automatique de la pompe à seringue afin de lancer l'administration du second fluide à partir de la nouvelle seringue par la pompe à seringue conformément au second ordre d'administration de fluide.

6. Système selon la revendication 5, dans lequel la commande de programmation automatisée amène la pompe à seringue à :
se programmer automatiquement pour lancer l'administration du premier fluide à partir de la première seringue par la pompe à seringue conformément au premier ordre d'administration de fluide ;
inviter un utilisateur, lorsque la première seringue est vidée, à décharger la première seringue et à charger la nouvelle seringue ; et
arrêter automatiquement l'administration du second fluide lorsque la quantité du second fluide administrée à partir de la nouvelle seringue satisfait la seconde portion du premier fluide restant à administrer.

7. Système selon l'une quelconque des revendications 2 à 6, dans lequel l'indication que le premier ordre d'administration de fluide est terminé comprend :
l'association d'un type du second fluide et d'un événement de fin de rinçage avec le premier ordre d'administration de fluide.

8. Système selon l'une quelconque des revendications 2 à 6, les opérations comprenant en outre : la réception, sur la base des données de capteur générées, d'une indication que la nouvelle seringue est vidée ; et la fourniture d'une indication que le second ordre d'administration de fluide est terminé lorsque l'indication de vidange de la nouvelle seringue est reçue.

9. Système selon l'une quelconque des revendications 1 à 8, les opérations comprenant en outre, lorsque la quantité du second fluide administrée à partir de la nouvelle seringue satisfait la seconde portion du premier fluide restant à administrer, automatiquement, sans intervention de l'utilisateur :
l'invitation d'un utilisateur à confirmer la poursuite de l'administration du second fluide à partir de la nouvelle seringue ; et
la poursuite de l'administration du second fluide à partir de la nouvelle seringue en réponse à la réception de la confirmation.

10. Support de stockage lisible par machine non transitoire incorporant des instructions qui, lorsqu'elles sont exécutées par une machine, amènent la machine à exécuter un procédé comprenant :
la réception, depuis un ou plusieurs capteurs associés à une pompe à seringue, d'une indication qu'une nouvelle seringue est chargée dans la pompe à seringue ;
la détermination, après réception de l'indication et sur la base d'informations stockées électroniquement associées à la pompe à seringue, que la pompe à seringue est actuellement associée à un premier ordre d'administration de fluide pour une quantité prédéterminée d'un premier fluide, et qu'au moins une première portion du premier fluide a été précédemment administrée par la pompe à seringue conformément au premier ordre d'administration de fluide avant que la nouvelle seringue ne soit chargée dans la pompe à seringue ;
la détermination d'une seconde portion du premier fluide restant à administrer pour compléter la quantité prédéterminée du premier fluide pour le premier ordre d'administration de fluide, la seconde portion du premier fluide se trouvant dans une ligne d'administration de fluide en aval ;
le lancement de l'administration d'un second fluide à partir de la nouvelle seringue par la pompe à seringue conformément à un second ordre d'administration de fluide, la pompe à seringue étant amenée à administrer le second fluide par l'entraînement d'un moteur de la pompe à seringue en réponse au lancement de l'administration du second fluide à partir de la nouvelle seringue, et poursuivant l'administration du second fluide à partir de la nouvelle seringue par l'entraînement du moteur de la pompe à seringue ; et
lorsqu'une quantité du second fluide administrée à partir de la nouvelle seringue satisfait la seconde portion du premier fluide restant à administrer, automatiquement, sans intervention de l'utilisateur et sans arrêter le moteur :
l'indication électronique que le premier ordre d'administration de fluide est terminé,
la dissociation du premier ordre d'administration de fluide de la pompe à seringue,
l'association de la pompe à seringue au second ordre d'administration de fluide,
la génération d'une condition de démarrage pour le second ordre d'administration, la condition de démarrage pour le second ordre d'administration de fluide n'étant générée qu'après que le premier ordre d'administration de fluide a été déterminé comme terminé du fait que la seconde portion restante a été purgée de la ligne d'administration de fluide en aval, et
la poursuite de l'administration du second fluide à partir de la nouvelle seringue.

11. Support lisible par machine non transitoire selon la revendication 10, le procédé comprenant en outre : la réception, avant que la nouvelle seringue ne soit chargée dans la pompe à seringue, d'une indication qu'une première seringue chargée dans la pompe à seringue est vidée ; la génération, sur la base de la réception de l'indication de vidange de la première seringue, d'une condition de vide pour le premier ordre d'administration de fluide ; la génération d'une condition de redémarrage pour le premier ordre d'administration de fluide simultanément au lancement de l'administration du second fluide conformément au second ordre d'administration de fluide ; et la génération d'une condition de démarrage pour le second ordre d'administration de fluide simultanément à l'association de la pompe à seringue au second ordre d'administration de fluide afin de poursuivre l'administration du second fluide à partir de la nouvelle seringue.

12. Support lisible par machine non transitoire selon la revendication 11, dans lequel l'administration du premier fluide et du second fluide est mesurée en unités volumétriques, le procédé comprenant en outre :
la mesure continue, pour le premier ordre d'administration de fluide, d'un volume total du premier fluide sur l'administration du premier fluide à partir de la première seringue avant la génération de la condition de vide et à travers l'administration du second fluide à partir de la nouvelle seringue jusqu'à ce que la quantité du second fluide administrée à partir de la nouvelle seringue satisfasse la seconde portion du premier fluide restant à administrer ;
la confirmation que le volume total mesuré du premier fluide a été administré conformément au premier ordre d'administration de fluide ; et
la fourniture du volume total pour affichage sur un dispositif d'affichage.

13. Support lisible par machine non transitoire selon la revendication 11 ou 12, dans lequel le premier fluide est un médicament et le second fluide est un liquide de rinçage non médicamenteux, le procédé comprenant en outre :
le fait de faire administrer par la pompe à seringue une portion du premier fluide à partir de la première seringue jusqu'à la réception de l'indication de vidange de la première seringue ;
la mise en pause du premier ordre d'administration de fluide avant le lancement de l'administration du second fluide, dans lequel, lorsque la première seringue est vidée et que l'administration du second fluide est lancée, la seconde portion du premier fluide restant à administrer réside dans une ligne de perfusion connectée à la pompe à seringue mais n'est pas administrée à un patient ; et
le fait de faire administrer par la pompe à seringue, en réponse au lancement de l'administration du second fluide, la portion du premier fluide depuis la ligne de perfusion en poussant la portion du premier fluide à travers la ligne de perfusion avec le second fluide.

14. Support lisible par machine non transitoire selon la revendication 13, le procédé comprenant en outre :
la réception, depuis un système informatique distant, d'une commande de programmation automatisée pour configurer la pompe à seringue afin d'administrer le liquide de rinçage non médicamenteux conformément au second ordre d'administration de fluide ; et
en réponse à la réception de la commande de programmation automatisée, sans intervention de l'utilisateur :
la désactivation d'une alarme de seringue vide actuellement configurée pour être fournie lorsque la première seringue chargée dans la pompe à seringue est vidée ; et
la programmation automatique de la pompe à seringue afin de lancer l'administration du second fluide à partir de la nouvelle seringue par la pompe à seringue conformément au second ordre d'administration de fluide.

15. Support lisible par machine non transitoire selon la revendication 14, dans lequel la commande de programmation automatisée amène la pompe à seringue à :
se programmer automatiquement pour lancer l'administration du premier fluide à partir de la première seringue par la pompe à seringue conformément au premier ordre d'administration de fluide ;
inviter un utilisateur, lorsque la première seringue est vidée, à décharger la première seringue et à charger la nouvelle seringue ; et
arrêter automatiquement l'administration du second fluide lorsque la quantité du second fluide administrée à partir de la nouvelle seringue satisfait la seconde portion du premier fluide restant à administrer.

16. Support lisible par machine non transitoire selon l'une quelconque des revendications 10 à 15, dans lequel l'indication que le premier ordre d'administration de fluide est terminé comprend :
l'association d'un type du second fluide et d'un événement de fin de rinçage avec le premier ordre d'administration de fluide.

17. Support lisible par machine non transitoire selon l'une quelconque des revendications 10 à 15, le procédé comprenant en outre :
la réception d'une indication que la nouvelle seringue est vidée ; et
la fourniture d'une indication que le second ordre d'administration de fluide est terminé lorsque l'indication de vidange de la nouvelle seringue est reçue.

18. Support lisible par machine non transitoire selon l'une quelconque des revendications 10 à 17, le procédé comprenant en outre, lorsque la quantité du second fluide administrée à partir de la nouvelle seringue satisfait la seconde portion du premier fluide restant à administrer, automatiquement, sans intervention de l'utilisateur :
l'invitation d'un utilisateur à confirmer la poursuite de l'administration du second fluide à partir de la nouvelle seringue ; et
la poursuite de l'administration du second fluide à partir de la nouvelle seringue en réponse à la réception de la confirmation.
